# EUROPEAN PATENT APPLICATION

(11) **EP 4 643 859 A1**
(43) Date of publication of application: **05.11.2025**
(21) Application number: 23927701.5
(22) Date of filing: 06.09.2023
(51) Int. Cl.: A61K 31/501, A61K 31/401, A61K 31/44, A61K 31/585, A61P 9/00, A61P 9/12

(54) **COMPOSITION FOR PREVENTING HEART DISEASES CAUSED BY PULMONARY HYPERTENSION AND TREATING HEART, AND FUNCTIONAL FORMULATION COMPRISING SAME**

(30) Priority: 16.03.2023 KR 20230034318
(71) Applicant: Careside Co., Ltd., Gyeonggi-do 13209 (KR)
(72) Inventor: YOU, Young Kook, Seongnam-si Gyeonggi-do 13586 (KR)
(74) Representative: Keenway Patentanwälte Neumann Heine Taruttis
(86) International application number: PCT/KR2023/013354
(87) International publication number: WO 2024/190980

(57) **Abstract**

There are provided a composition for preventing and treating heart disease due to pulmonary hypertension, and a functional preparation containing the same. The composition includes pimobendan, enalapril, torsemide, and spironolactone, and is thus effective in preventing and treating heart disease and pulmonary hypertension of animals.

## Description

### [Technical Field]

The present disclosure relates to a composition for preventing and treating heart disease and a functional preparation containing the same. More particularly, the present disclosure relates to a composition for preventing and treating heart disease such as heart failure due to pulmonary hypertension in animals such as dogs and cats rather than humans, and a functional preparation for animals containing the same.

### [Background Art]

The heart serves to receive blood from veins and continuously circulate blood throughout the body through arteries. The heart contracts in order to deliver the blood, and the cardiac muscle composed of muscles causes such contraction. In this regard, when a ventricular load increases due to hypertension or heart valve disease or myocardial cells themselves are damaged due to myocardial infarction, myocarditis, or cardiomegaly, an amount of blood to the organs of the body may not be sufficiently pumped, resulting in a decrease in cardiac output and cardiac hypertrophy. The heart is an organ whose differentiation has been completely completed embryologically, and no more cell proliferation is possible in the heart. Accordingly, when it becomes necessary to increase cardiac output, the only solution is to increase the contractility of the cardiac muscle by increasing sizes of existing cardiac muscle cells, and such a physiological phenomenon is called hypertrophy. When such hypertrophy continues for a certain period, there is a very high possibility that it may worsen into heart failure.

### [Disclosure]

### [Technical Problem]

One of the causes of heart failure is pulmonary hypertension. When the blood passes from the right heart to the lungs through the pulmonary artery, carbon dioxide in the blood is removed and oxygen is supplied. Here, when a blood pressure in the pulmonary artery rises to a high level, this status is called pulmonary hypertension, and in this case, it may become difficult to send blood from the right heart to the pulmonary artery. Accordingly, the right ventricle of the heart thickens and hypertrophies, which may cause right heart failure.

Aspects of the present disclosure provide a composition effective in preventing and treating heart disease such as heart failure and a functional preparation containing the same.

Aspects of the present disclosure also provide a composition effective in preventing and treating heart disease such as heart failure and pulmonary hypertension which may be a cause of the heart disease, and a functional preparation containing the same.

However, aspects of the present disclosure are not restricted to those set forth herein. The above and other aspects of the present disclosure will become more apparent to one of ordinary skill in the art to which the present disclosure pertains by referencing the detailed description of the present disclosure given below.

### [Technical Solution]

According to an aspect of the present disclosure, there are provided a composition and a functional preparation for preventing and treating heart disease and pulmonary hypertension of an animal, including: pimobendan; enalapril; torsemide; and spironolactone.

The composition and the functional preparation may include 0.5 mg of pimobendan, 1 mg of enalapril, 0.2 mg of torsemide, and 2 mg of spironolactone.

Based on a weight of a target, a single administration dose of pimobendan may be 0.25 mg/kg, a single administration dose of enalapril may be 0.5 mg/kg, a single administration dose of torsemide may be 0.1 mg/kg, and a single administration dose of spironolactone may be 1 mg/kg, and pimobendan, enalapril, torsemide, and spironolactone may be administered twice a day.

The target may be a dog or a cat.

Detailed contents of other embodiments are described in a detailed description.

### [Advantageous Effects]

A composition according to embodiments of the present disclosure is effective in preventing and treating heart disease and pulmonary hypertension of animals by including pimobendan, enalapril, torsemide, and spironolactone.

The composition according to the present disclosure may be produced into preparations of various dosage forms regardless of formulation, and may also be utilized as an additive to various preparations.

The composition according to embodiments of the present disclosure is effective for pets such as dogs and cats, and may be produced into snacks and foods for pets.

The effects of the present disclosure are not limited to the aforementioned effects, and various other effects are included in the present specification.

### [Description of Drawings]

FIGS. 1 to 5 are graphs illustrating results of a clinical test of Example 1;
FIGS. 6 to 8 are graphs illustrating evaluation results of a blood index test of Example 1;
FIGS. 9 to 14 are graphs illustrating evaluation results of a serum biochemical index test of Example 1;
FIGS. 15 to 18 are graphs illustrating evaluation results of a serum electrolyte index of Example 1;
FIGS. 19 to 21 are graphs illustrating results of a biomarker test of Example 1;
FIGS. 22 to 24 are graphs illustrating results of a chest radiograph evaluation of Example 1;
FIGS. 25 to 32 are graphs illustrating results of a cardiac ultrasound evaluation of Example 1;
FIG. 33 is a graph illustrating an evaluation result of an international small animal cardiac heart council (ISACHC) stage of Example 1;
FIGS. 34 to 38 are graphs illustrating results of a clinical test of Example 2;
FIGS. 39 to 41 are graphs illustrating results of a chest radiograph evaluation of Example 2;
FIGS. 42 to 45 are graphs illustrating results of a cardiac ultrasound evaluation of Example 2;
FIG. 46 is a graph illustrating a severity evaluation result of pulmonary hypertension of Example 2; and
FIG.47 is a graph illustrating an evaluation result of an ISACHC stage of Example 2.

### [Mode for Invention]

Advantages and features of the present disclosure and methods for accomplishing these advantages and features will become apparent from embodiments to be described later in detail with reference to the accompanying drawings. However, the present disclosure is not limited to embodiments disclosed below, and may be implemented in various different forms, these embodiments will be provided only in order to make the present disclosure complete and allow one of ordinary skill in the art to which the present disclosure pertains to completely recognize the scope of the present disclosure, and the present disclosure will be defined by the scope of the claims.

In addition, 'and/or' includes each and all combinations of one or more of the mentioned items. A numerical range indicated using 'to' indicates a numerical range including values described before and after 'to' as a lower limit and an upper limit, respectively. 'About' or 'approximately' refers to a value or a numerical range within 20% of a value or a numerical range described after 'about' or 'approximately'.

Hereinafter, embodiments of the present disclosure will be described.

A composition for preventing and treating heart disease according to an embodiment of the present disclosure includes pimobendan, enalapril, torsemide, and spironolactone. The composition according to the present disclosure is effective in preventing and treating heart disease in animals such as dogs and cats rather than humans. In particular, the composition according to the present disclosure may be effective in preventing and treating diseases in which a heart size changes, such as heart failure among heart diseases, and pulmonary hypertension, which is one of the causes of such diseases.

"Heart failure" as used herein refers to any contractile disorder or heart disease. In general, clinical symptoms are a result of changes in cells and molecular components of the heart and changes in mediators driving homeostatic control. In general, the heart failure is related to an increase in heart size and deterioration of a cardiac function.

Pimobendan is an inodilator compound having a calcium-sensitizing effect and several phosphodiesterase type III inhibitory effects. A calcium-sensitizing agent achieves a positive inotropic effect by sensitizing contractile proteins to cytosolic calcium to alter the binding of calcium to troponin-C, rather than increasing calcium flowing into myocardial cells. By achieving the positive inotropic effect through calcium sensitization, some of the side effects of cytosolic calcium overload are prevented. An increase in cytosolic calcium level is related to an increase in tendency to arrhythmias and sudden death. Clinical tests in which dogs with heart failure take oral pimobendan for a long period have demonstrated that exercise tolerance and quality of life were improved without a significant side effect on survival.

Pimobendan is an inodilator having both positive myocardial force-increasing and vessel relaxation effects. Such effects result in a cardiotonic effect by increasing the sensitivity of contractile apparatuses in myocardial cells to calcium in addition to a PDEi-3 effect, and thus, pimobendan is also called a calcium sensitizer. Since pimobendan exerts the cardiotonic effect without increasing myocardial oxygen demand, pimobendan is a drug that does not aggravate an ischemic change in the cardiac muscle in heart failure, and increases myocardial contractility with almost no risk of arrhythmia unlike digitalis. Pimobendan is indicated for chronic treatment in all dogs with heart failure secondary to chronic mitral vavular insufficiency (CMVI) or dilated cardiomyopathy (DCM). Pimobendan has been demonstrated to be very beneficial in the treatment of dogs with clinical symptoms of heart failure and be excellent in improving clinical symptoms and prolonging survival (PITCH study 2000 and QUEST study 2008). A therapeutic effect of pimobendan was found to be more effective in dogs with CMVI and DCM. The EPIC study (2018) investigated whether or not there were an improvement in clinical/imaging diagnosis findings and a difference in disease progression when pimobendan was administered to dogs with subclinical (asymptomatic) CMVI. In this study, it was confirmed that LVIDd and LA/Ao was decreased on imaging diagnosis at 35 days of administration in a pimobendan administered group compared to a placebo administered group.

Enalapril is a drug used to treat hypertension, diabetic kidney disease, and heart failure. In the case of the heart disease, enalapril is generally used together with a diuretic such as furosemide. Enalapril may be orally or intravenously administered, and an effect of enalapril usually occurs within 1 hour and lasts up to a day when enalapril is orally taken. Enalapril belongs to the ACE inhibitor class of drugs, and general side effects of enalapril may include headache, fatigue, and cough.

Angiotensin converting enzyme inhibitors (ACEi), such as enalapril cause vasodilation by inhibiting the formation of angiotensin II, which is a vasoconstriction factor produced by an action of renin secreted by the kidneys. Clinically, the ACEi is mainly used to treat heart failure, hypertension secondary to kidney disease, and proteinuria. Efficacy of various ACEi (enalapril, benazapril, imidapril, and captopril) has been evaluated in dogs with clinical symptoms (COVE study-1995; Live study-1998; and BENCH trial-1999). These studies have demonstrated that ACEi administration to dogs with heart failure due to DCM and CMVI slows the progression of heart failure, improves clinical symptoms, and prolongs survival.

Torsemide is a diuretic used to treat fluid overload due to heart failure, kidney disease, liver disease, and hypertension. Torsemide is an ingredient that is not generally preferred for the treatment of hypertension itself, is a loop diuretic such as sulfonamide, and has an effect of inhibiting sodium reabsorption in the kidneys. General side effects of torsemide may include headache, an increase in amount of urine, diarrhea, cough, and dizziness, and other side effects of torsemide may include hearing loss and hypokalemia.

A diuretic is the most commonly used and effective treatment drug for treating heart failure. The diuretic reduces a circulating blood volume to reduce venous return, and thus, ultimately reduces an amount of blood (full load) flowing into the ventricle. A diuretic mainly used in small animal clinical practice includes (1) a loop diuretic, (2) a thiazide diuretic, (3) a potassium-sparing diuretic, and the like. Among them, a diuretic having the strongest diuretic effect is the loop diuretic such as torsemide. Furosemide is traditionally the most widely used drug in a veterinary medicine field, but it shows a ceiling effect (a phenomenon in which the efficacy is halved after a predetermined time has elapsed) and has lower bioavailability than torsemide when it is orally administered. In a study investigating pharmacokinetic characteristics of torsemide in dogs, torsemide showed almost no ceiling effect and showed continuous efficacy even with long-term administration. In addition, in a comparative dosing clinical study RCT of two drugs recently conducted in France, torsemide showed excellent efficacy. In dogs that are refractory to administration of furosemide, torsemide is usually indicated, but because torsemide has high bioavailability, a long blood half-life, and almost no ceiling effect, a method of administering torsemide instead of furosemide is advantageous in managing dogs with heart failure without increasing a diuretic dose.

Spironolactone is one of anti-aldosterone agents, potassium-sparing diuretics, and spiro compounds, and shows a diuretic effect in which potassium ions are preserved in the body by inhibiting the binding of aldosterone to its receptors in the body. Spironolactone is mainly used for the treatment of heart failure, cirrhotic ascites, and hypertension, and may also be used as an adjuvant treatment drug for hypokalemia.

A potassium-sparing diuretic such as spironolactone does not directly act on sodium transport, and antagonizes an action of aldosterone in a collecting duct unlike other diuretics. For this reason, reabsorption of sodium (water) is blocked to cause diuresis. However, since excretion of potassium and hydrogen ions does not occur in this area, there is no change in a potassium or hydrogen concentration in the blood. For this reason, this type of diuretic is called a potassium-sparing diuretic. However, since a sodium absorption amount in this area accounts for a very low proportion of total sodium reabsorption, a diuretic effect is very low (~10% of the efficacy of the loop diuretic). Accordingly, spironolactone is not used alone, and is often used in combination with the thiazide diuretic or the loop diuretic (in order to prevent hypokalemia). A study on whether or not spironolactone slows the progression of heart failure was conducted on dogs with subclinical myxomatous mitral valve disease (MMVD), but the effect was not obviously proven. However, an increase in heart size and cardiac biomarker concentration on cardiac ultrasound was much smaller in a spironolactone-administered group than in a placebo-administered group.

According an embodiment, the composition according to the present disclosure may include 5 parts by weight of pimobendan, 10 parts by weight of enalapril, 2 parts by weight of torsemide, and 20 parts by weight of spironolactone. The composition according to the present disclosure may include pimobendan, enalapril, torsemide, and spironolactone in amounts effective in preventing and treating the heart disease, respectively, and may include the respective ingredients in amounts required for a single administration in consideration of a body weight of a target group to which the composition according to the present disclosure is administered, for example, a target such as a dog or a cat. According to an embodiment, the composition may include 0.5 mg of pimobendan, 1 mg of enalapril, 0.2 mg of torsemide, and 2 mg of spironolactone. A tablet prepared by tableting the composition may include 0.5 mg of pimobendan, 1 mg of enalapril, 0.2 mg of torsemide, and 2 mg of spironolactone per tablet. The total weight range of one tableted tablet is not limited, and may be set in consideration of a type, medication taking ability, and absorption rate of ingredients of the tablet of dog. As an example, the tablet prepared by tableting the composition may have a weight of 100 mg per tablet, and may include 0.5 mg of pimobendan, 1 mg of enalapril, 0.2 mg of torsemide, and 2 mg of spironolactone per tablet. For a detailed description thereof, refer to experimental examples to be described later.

The composition for preventing and treating heart disease according to embodiments of the present disclosure may be prepared in various oral or parenteral dosage forms. When the composition is formulated, it is prepared using diluents or excipients such as fillers, extenders, binders, wetting agents, disintegrating agents, and surfactants that are usually used. Solid preparations for oral administration include tablets, pills, powders, granules, capsules, or the like, and are prepared by mixing one or more excipients such as starch, calcium carbonate, sucrose or lactose, and gelatin with one or more compounds. In addition to simple excipients, a lubricant such as magnesium stearate and talc is also used. Liquid preparations for oral administration are suspensions, oral liquids, emulsions, syrups, and the like, and may include several excipients such as wetting agents, sweeteners, flavoring agents, and preservatives in addition to water or liquid paraffin, which is a commonly used simple diluent. Preparations for parenteral administration include sterile aqueous solutions, non-aqueous solvents, suspensions, emulsions, lyophilizers, and suppositories. As the non-aqueous solvents and the suspensions, propylene glycol, polyethylene glycol, vegetable oil such as olive oil, and injectable esters such as ethyl oleate, and the like, may be used. As a base of the suppository, witepsol, macrogol, Tween 61, cacao butter, laurin butter, glycerogelatin, or the like, may be used.

According to an embodiment, the composition for preventing and treating heart disease may be formulated by including excipients. For example, the composition for preventing and treating heart disease may be prepared as a tablet by including fats such as chicken fat and tocopherol acetate. Since the fats and tocopherol acetate are coated on a surface of the tablet, the fats are not absorbed up to an inner portion of the tablet, such that it is possible to prevent the tablet from being broken or becoming soggy.

The composition for preventing and treating heart disease according to embodiments of the present disclosure may be prepared as various functional foods. For example, the composition for preventing and treating heart disease according to embodiments of the present disclosure may be prepared as various foods such as chewing gum, caramel products, candies, ice cream, and confectionery, beverages such as soft drinks, mineral water, and alcoholic beverages, and health functional foods such as vitamins or minerals. In addition, the composition for preventing and treating heart disease according to embodiments of the present disclosure may be formulated into an oral preparation for pets, specifically, a tablet, a capsule, liquid, gel, paste, oral spray, oral tablet, powder, and chewable treat for pets, or animal feed, but is not limited thereto.

The composition for preventing and treating heart disease according to embodiments of the present disclosure may be prepared as a food as it is or used together with other food ingredients, and may be appropriately used according to a general method. Amounts of mixed active ingredients may be appropriately determined according to a use purpose.

The functional food according to the present disclosure may contain various flavoring agents or a natural carbohydrate as an additional ingredient. The above-described natural carbohydrate is monosaccharide such as glucose and fructose or a sugar alcohol such as sorbitol and erythritol. As the sweetener, a natural sweetener such as thaumatin and stevia extract, or a synthetic sweetener such as saccharin and aspartame may be used. A ratio of the natural carbohydrate is preferably selected in the range of 0.01 to 0.04 parts by weight, preferably about 0.02 to 0.03 parts by weight per 100 parts by weight of the health functional food according to the present disclosure.

The functional food according to the present disclosure may contain various nutrients, vitamins, electrolytes, flavoring agents, coloring agents, pectic acid and its salts, alginic acid and its salts, organic acids, protective colloid thickeners, pH regulators, stabilizers, preservatives, glycerin, alcohol, carbonating agents used in carbonated beverages, and the like, in addition to the above-described ingredients. In addition, the functional food according to the present disclosure may contain fruit pulp for the preparation of natural fruit juice, fruit juice beverages, and vegetable beverages. These ingredients may be used independently or in combination. A ratio of such additives is not important, but is generally selected in the range of 0.01 to 0.1 parts by weight per 100 parts by weight of the functional food according to the present disclosure.

Hereinafter, the present disclosure will be described in more detail through Examples. Such Examples are provided only to describe the present disclosure, and the scope of the present disclosure is not limited by such Examples.

### <Example 1> Preparation of composition for preventing and treating heart disease

A composition for treating heart disease was prepared by mixing compositions in Table 1 with each other. A unit in Table 1 is mg. A tablet having a content of 100 mg per tablet was prepared by tableting a composition having the following composition.

**[Table 1]**

| Ingredient | Content (100 mg) |
|---|---|
| Pimobendan | 0.5mg |
| Enalapril | 1mg |
| Torsemide | 0.2mg |
| Spironolactone | 2mg |
| Preservative | Appropriate amount |
| Other additive | Appropriate amount |

### <Experimental Example 1> Confirmation of heart disease prevention and treatment effects

The following experiment was performed in order to confirm heart disease prevention and treatment effects by the composition of Example 1.

### <Experimental Method>

A test group to which the composition of Example 1 is to be administered and a placebo group to which a placebo is to be administered were selected, and an experiment was performed to evaluate the efficacy and stability according to administration. As the test group and the placebo group, 30 dogs, which are experimental animals diagnosed with heart failure of an international small animal cardiac heart council (ISACHC) stage III or higher, were selected. The 30 experimental animals were selected as the same gender compositions of small dog breeds and 15 females and 15 males.

Heart failure statuses of the selected experimental animals were classified according to direct examination by a veterinary cardiologist through a clinical test, a chest radiograph test, and a cardiac ultrasound test, and an ACVIM pulmonary hypertension guideline. Dogs with other organ diseases in the clinical test were excluded.

As a test drug administration method, the tablet prepared in Example 1 was orally administered to the experimental animal twice a day, and 1/2 tablet per weight of 1 kg of the experimental animal was administered when it was administered once. The tablet was administered at 12-hour intervals in the morning and evening at the same time regardless of meals, and an administration period was 56 days.

In a plan of the present experiment, an administration experiment was performed on dogs with heart failure caused by various causes including valvular disease. ISACHC stage III heart failure is a severe heart failure status, a group of dogs with the ISACHC stage III heart failure is a group with obvious clinical symptoms of heart disease such as pulmonary edema or faint, and pulmonary hypertension indices of dogs with pulmonary hypertension as complications of heart disease among such dogs were separately recorded, and improvement effects according to drug administration were also investigated.

Test intervals for drug administration were a day before administration (DAY 0), 7 days of administration (DAY 7), 14 days of administration (DAY 14), 28 days of administration (DAY 28), and 56 days of administration (DAY 56).

Specific observation items and test method are as follows.

### Observation items

1) CBC: Hematological indices (RBC index and WBC index)
2) Serum biochemical test: renal indices (BUN and creatinine), liver indices (ALT and ALP), total protein (TP, albumin), electrolyte indices (Na, K, Cl, P, etc.)
3) Evaluation of heart and lung field on radiograph: VHS, VLAS, lung field pattern
4) Cardiac ultrasound: LA/Ao, LVIDdn, MVE, E/E', TR velocity, PR velocity, RVOT ET/AT, RVOT AT measurement
5) Blood symmetric dimethyl arginine (SDMA) concentration: evaluation of improvement of kidney numerical value
6) Blood N-terminal pro-brain natriuretic peptide (NT-proBNP) concentration: evaluation of improvement of heart numerical value
7) Blood canine pancreatic lipase immunoactivity (cPL) concentration: evaluation of improvement of pancreatic numerical value
8) As clinical test items, exercise intolerance (1 to 4; 1 is best and 4 is worst), appetite (1 to 4), respiratory effort (1 to 4), cough (1 to 4), and faint (1 to 4) were checked with different numerical values, respectively.

As the observation test method, tests for clinical test items were performed on a day before administration (DAY 0), 7 days of administration (DAY 7), 14 days of administration (DAY 14), 28 days of administration (DAY 28), and 56 days of administration (DAY 56), respectively, and test results were recorded by a veterinarian and guardian, respectively. Tests for radiograph, cardiac ultrasound, and biomarker, which are the observation items, were performed at designated hospitals and test results were recorded.

Effect evaluation criteria according to test results for the respective clinical test items were as follows.

### 1) Effect evaluation criteria

- Whether or not clinical symptoms are improved: increase in vitality , decrease in the number of times of cough, increase in appetite, decrease in respiratory effort, decrease in the number of times of faint
- Improvement of CBC and serum biochemical indices
- Improvement of heart size and lung field infiltration status on chest radiograph
- Improvement of cardiac test indices on cardiac ultrasound
- Improvement findings on cardiac biomarker test

### 2) Effect evaluation method

- Whether or not clinical symptoms are improved: evaluated through guardian records and veterinarian examination records
- Improvement of CBC and serum biochemical indices: evaluated for liver, kidney, pancreas, and electrolyte test items
- Relief of pulmonary hypertension on chest radiograph: determined through evaluation of VHS (heart size), VLAS (left atrial size), and lung field pattern (lung field infiltration findings)
- Improvement of cardiac test indices on cardiac ultrasound: determined through evaluation of LA/Ao (aorta to left atria diameter ratio), LVIDdn (left ventricular end-diastolic diameter), MVE (bicuspid valve filling velocity), E/E' (bicuspid valve filling velocity to tissue Doppler velocity ratio), TR velocity (tricuspid value regurgitation velocity), PR velocity (pulmonary artery regurgitation velocity), RVOT ET/AT (right ventricular outflow tract ejection time/acceleration time), and RVOT AT (right ventricular outflow tract acceleration time)
- Improvement findings on cardiac biomarker test: Renal circulation improvement and renal remodeling inhibition degrees were evaluated through a concentration measurement of SDMA indicating a renal perfusion status, and it was evaluated whether or not a right heart pressure is decreased due to pulmonary hypertension was evaluated through an NT-proBNP concentration measurement

### 3) Statistical interpretation method

- Normal distribution of each test index was tested through the Kolmogorov-Smirnov test.
- A difference in test indices between respective groups was compared through the Mann-Whitney U test, and the Wilcoxson signed rank test was performed for a comparison before and after administration.
- P < 0.05 was evaluated as being statistically significant.

### <Experimental Result 1>

### 1. Clinical test

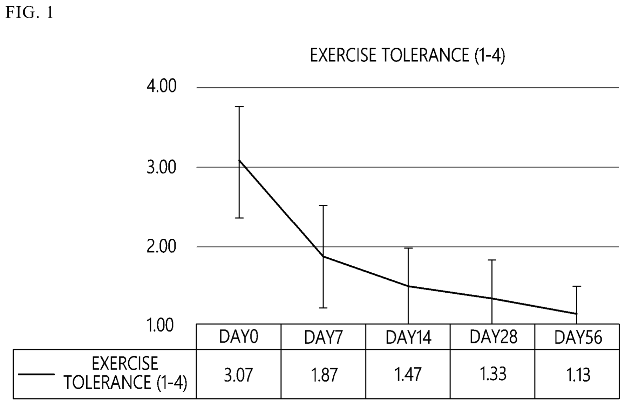

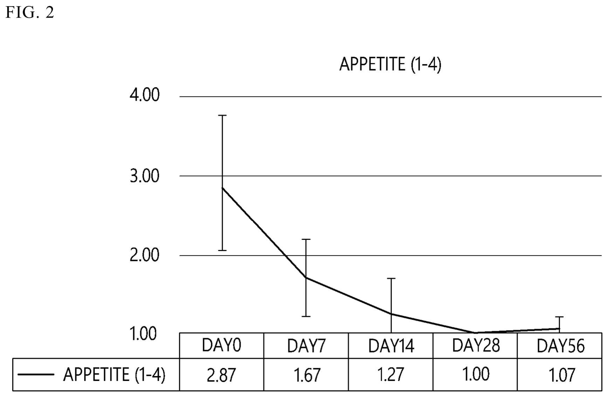

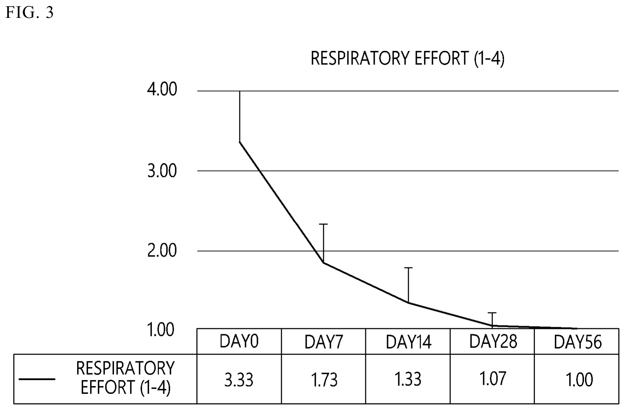

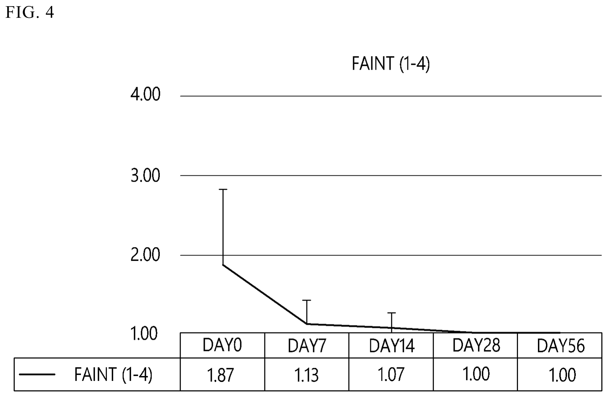

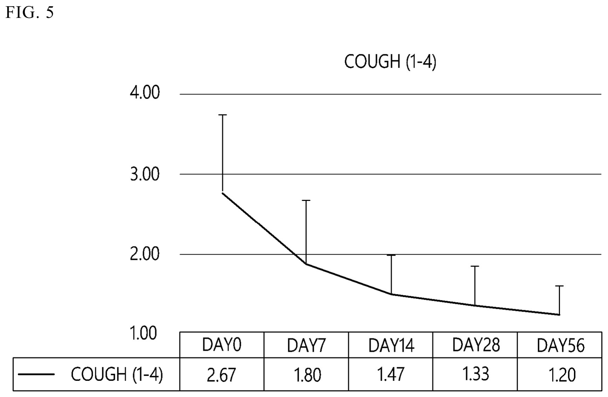

As results of a clinical test of Example 1, exercise intolerance, appetite, faint, respiratory effort, and cough evaluation results were illustrated in Table 2 and FIGS. 1 to 5.

**[Table 2]**

| Test index /Test point in time | DAY 0 | DAY 7 | DAY 14 | DAY 28 | DAY 56 |
|---|---|---|---|---|---|
| Exercise intolerance (1 to 4) | 3.07 | 1.87 | 1.47 | 1.33 | 1.13 |
| Appetite (1 to 4) | 2.87 | 1.67 | 1.27 | 1.00 | 1.07 |
| Faint (1 to 4) | 3.33 | 1.73 | 1.33 | 1.07 | 1.00 |
| Respiratory effort (1 to 4) | 2.67 | 1.80 | 1.47 | 1.33 | 1.20 |
| Cough (1 to 4) | 1.87 | 1.13 | 1.07 | 1.00 | 1.00 |

In Table 1, numerical values 1 to 4 of clinical evaluation results were evaluated as indicating the following cases, respectively.
- Exercise intolerance: 1. None at all, 2. Feels tired after a walk for a long time, 3. Has difficulty in walking during a walk, 4. Refuses to walk
- Appetite: 1. Very good, 2. Good, 3. Picky eater (Tries to eat only delicious food), 4. Refuses to eat food
- Respiratory effort: 1. None at all, 2. Hyperventilates after having fun, 3. Hyperventilates within 30 minutes even during a rest, 4. Hyperventilates even during sleep
- Faint: 1. None at all, 2. Faints once or less a day after exercise or excitement, 3. Faints once or less a day regardless of exercise or excitement, 4. Faints once or more a day regardless of exercise or excitement
- Cough: 1. None at all, 2. Coughs when excited or drinking water, 3. Coughs less than 10 times a day regardless of excitement or drinking water, 4. Cannot sleep because of cough

FIGS. 1 to 5 are graphs illustrating results of a clinical test of Example 1. FIGS. 1 to 5 illustrate exercise intolerance, appetite, faint, respiratory effort, and cough evaluation results of each of the test group and the placebo group, respectively, as the results of the clinical test.

Referring to FIGS. 1 to 5, in the case of an exercise intolerance evaluation, exercise intolerance of sick dogs was significantly improved from a week of administration (DAY 7), and accordingly, these dogs did not feel particular fatigue during their daily lives. From 14 days of administration (DAY 14), most of the sick dogs did not show exercise intolerance symptoms in their daily lives.

In the case of an appetite evaluation, appetites of sick dogs were significantly improved from a week of administration (DAY 7), and accordingly, these dogs did not particularly refuse food during their daily lives, and from 14 days of administration (DAY 14), most of the sick dogs showed healthy appetites in their daily lives.

In the case of a respiratory effort evaluation, effortful respiratory symptoms of sick dogs were significantly improved from a week of administration (DAY 7), and accordingly, these dogs did not have significant problems in their daily lives. From 14 days of administration (DAY 14), most of the sick dogs did not show special effortful respiratory symptoms in their daily lives.

In the case of a cough evaluation, cough symptoms of sick dogs were significantly improved from a week of administration (DAY 7), and accordingly, these dogs did not have significant problems in their daily lives. From 14 days of administration (DAY 14), most of the sick dogs did not show cough symptoms unless they were stimulated in their daily lives.

In the case of a faint evaluation, sick dogs hardly showed faint symptoms from a week of administration (DAY 7), and sick dogs showing faint symptoms again were hardly observed within other administration periods.

### 2. Blood index test

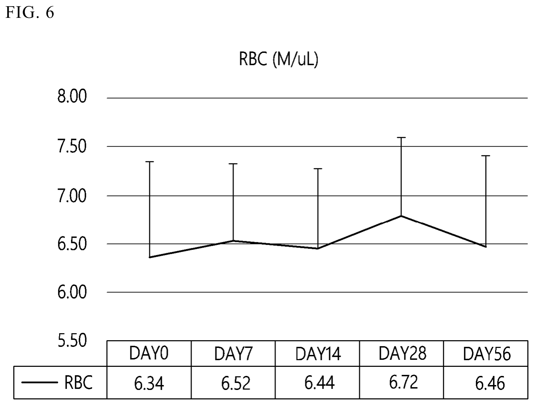

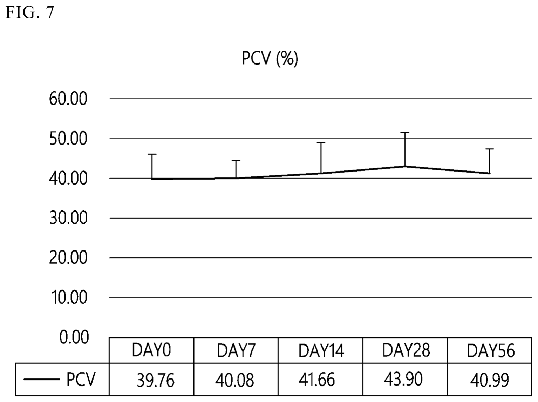

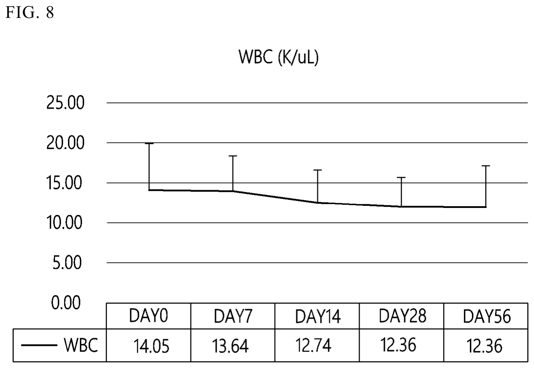

As a blood index test of Example 1, anemia or polycythemia (occurring in response to cyanosis) was evaluated through an evaluation of red blood cell count (RBC) and hematocrit (PCV) during the administration periods. In addition, it was evaluated whether or not inflammation occurred by evaluating a white blood cell count (WBC). Evaluation results of the blood index test were illustrated in Table 3 and FIGS. 6 to 8.

**[Table 3]**

| Test index /Test point in time | DAY 0 | DAY 7 | DAY 14 | DAY 28 | DAY 56 |
|---|---|---|---|---|---|
| RBC | 6.34 | 6.52 | 6.44 | 6.72 | 6.46 |
| PCV | 39.76 | 40.08 | 41.66 | 43.90 | 40.99 |
| WBC | 14.05 | 13.64 | 12.74 | 12.36 | 12.36 |

FIGS. 6 to 8 are graphs illustrating evaluation results of a blood index test of Example 1. FIGS. 6 to 8 illustrate evaluation results of red blood cell count (RBC), hematocrit (PCV), and white blood cell count (WBC) during the administration periods, respectively.

Referring to Table 3 and FIGS. 6 to 8, obvious changes in blood cell ratio were not observed before and after administration.

### 3. Serum biochemical index test

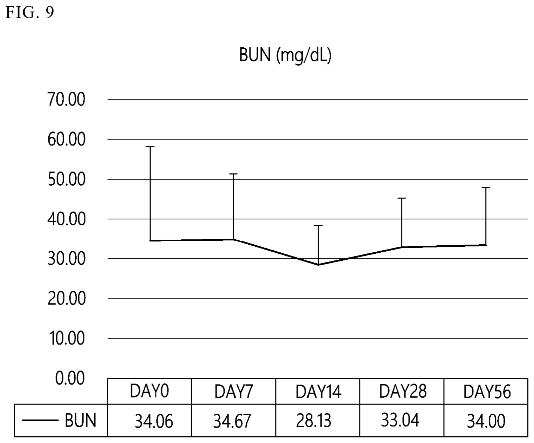

As a serum biochemical index test of Example 1, biochemical indices (BUN and creatinine) for a renal function, biochemical indices (ALT and ALP) for a liver function, and indices (TP and albumin) for serum protein were evaluated during administration periods. Evaluation results of the serum biochemical index test were illustrated in Table 4 and FIGS. 9 to 14.

**[Table 4]**

| Test index /Test point in time | DAY 0 | DAY 7 | DAY 14 | DAY 28 | DAY 56 |
|---|---|---|---|---|---|
| BUN | 34.06 | 34.67 | 28.13 | 33.04 | 34.00 |
| Creatinine | 1.12 | 1.10 | 1.06 | 1.15 | 1.13 |
| ALP | 170.47 | 172.73 | 185.20 | 198.93 | 136.47 |
| ALT | 78.93 | 79.47 | 66.80 | 55.80 | 59.27 |
| TP | 6.18 | 6.38 | 6.53 | 6.55 | 6.67 |
| Alb | 3.01 | 3.05 | 3.11 | 3.12 | 3.15 |

FIGS. 9 to 14 are graphs illustrating evaluation results of a serum biochemical index test of Example 1. FIGS. 9 to 14 illustrate evaluation results of BUN, creatinine, ALP, ALT, TP, and albumin (Alb), respectively.

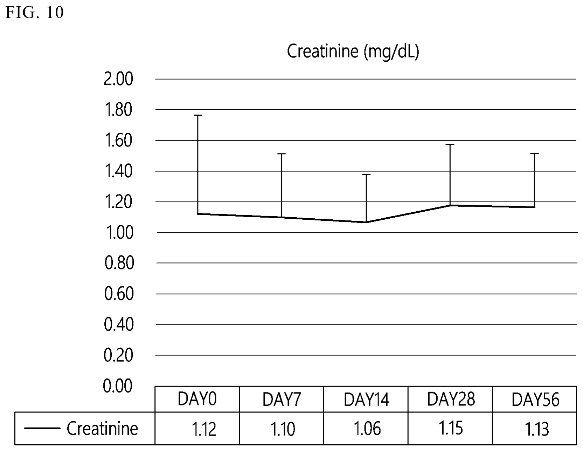

Referring to Table 4 and FIGS. 9 to 14, it was reported that when an excessive amount of heart medicine (e.g., diuretic) is administered, an increase in renal indices is generally observed, but as in the evaluation result of BUN illustrated in FIG. 9 and the evaluation result of creatinine illustrated in FIG. 10, an obvious change in renal indices was not observed before and after administration of Example 1 including the composition according to the present disclosure.

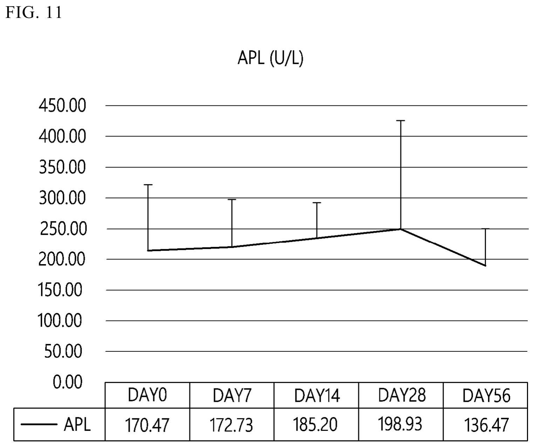

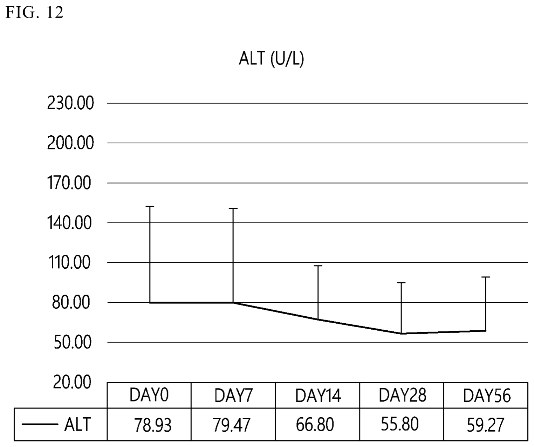

An increase in liver enzyme level in dogs with right heart failure (pulmonary hypertension) has been widely reported. It has been reported that such an increase in liver enzyme level is alleviated when a heart failure symptom is improved. It was observed that liver enzyme test indices are gradually improved (decreased) before and after administration also in the present experiment, as in the evaluation result of ALP illustrated in FIG. 11 and the evaluation result of ALT illustrated in FIG. 12.

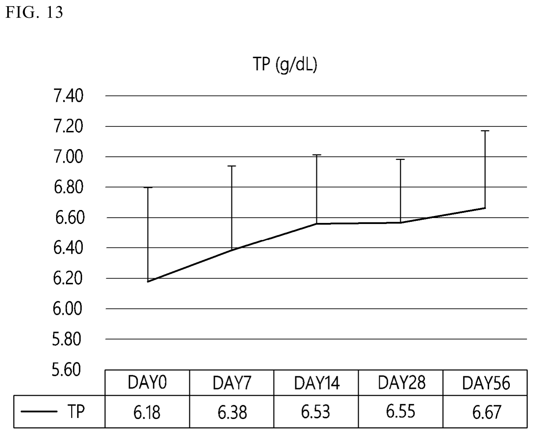

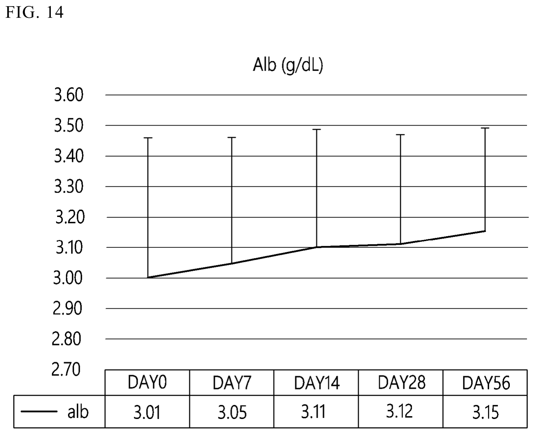

Total protein and albumin shows a tendency to increase in accordance with administration of an excessive amount of diuretic included in a cardiac drug (when dehydration is too severe), but shows a tendency to decrease when a heart failure symptom such as a decrease in liver function or fluid retention is severe. As in the evaluation result of TP in FIG. 13 and the evaluation result of Alb in FIG. 14, in the present experiment, these serum protein indices showed a tendency to gradually increase after administration. However, a severe increase suggesting dehydration was not observed, and only a gradual increase tendency due to suppression of fluid retention and liver treatment was observed.

### 4. Serum electrolyte index test

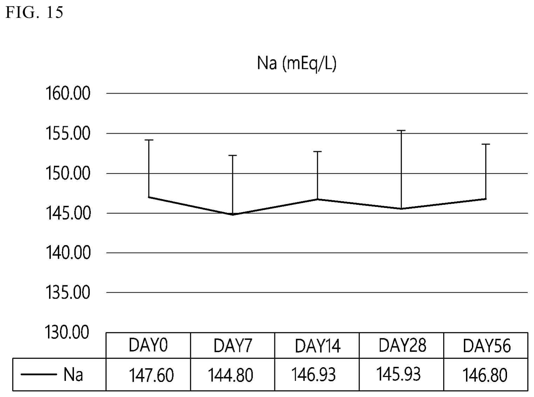

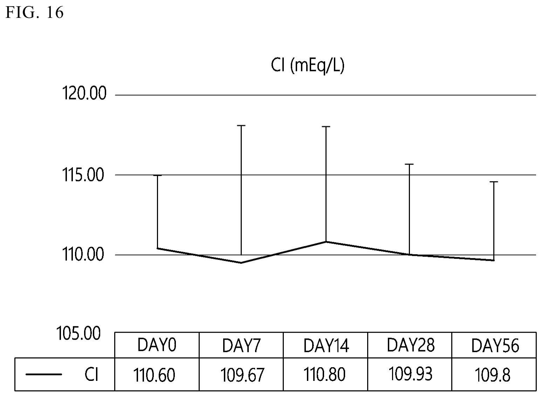

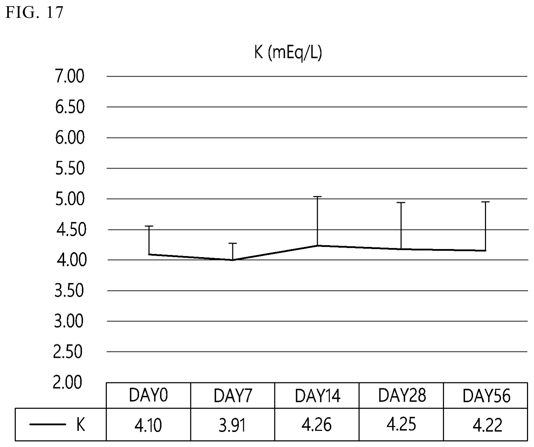

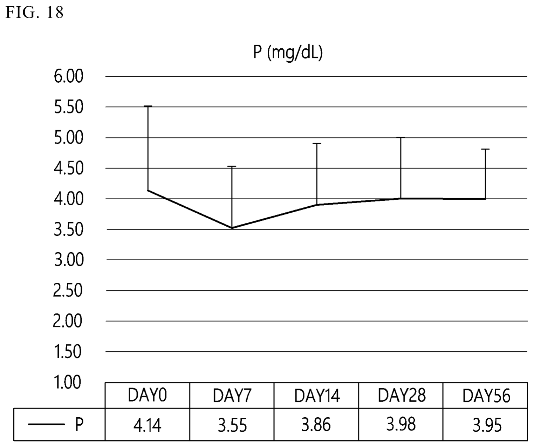

As a serum electrolyte index test of Example 1, changes in concentrations of Na, K, Cl, and P, which are electrolytes in a serum, were evaluated during administration periods. The results were illustrated in Table 5 and FIGS. 15 to 18.

**[Table 5]**

| Test index /Test point in time | DAY 0 | DAY 7 | DAY 14 | DAY 28 | DAY 56 |
|---|---|---|---|---|---|
| Na | 147.60 | 144.80 | 146.93 | 145.93 | 146.80 |
| Cl | 110.60 | 109.67 | 110.80 | 109.93 | 109.8 |
| K | 4.10 | 3.91 | 4.26 | 4.25 | 4.22 |
| P | 4.14 | 3.55 | 3.86 | 3.98 | 3.95 |

FIGS. 15 to 18 are graphs illustrating evaluation results of a serum electrolyte index of Example 1. FIGS. 15 to 18 illustrate changes in concentrations of Na, Cl, K, and P, which are serum electrolytes, in blood, respectively.

Due to characteristics of a cardiac drug, a side effect frequently occurring in association with cardiac drug administration is electrolyte imbalance. Torsemide of the ingredients included in the present test drug acts on the renal Henley loop to remove sodium and exert its pharmacological effect, and thus, hyponatremia, hypokalemia, and hypochloremia occur. On the other hand, drugs such as spironolactone or enalapril act on the collecting duct to retain potassium, and thus, hyperkalemia or hypernatremia may occur. Accordingly, when these drugs are appropriately mixed with each other, such electrolyte imbalance may be prevented.

Referring to Table 5 and FIGS. 15 to 18, also in the present experiment, before and after administration, statistically meaningful changes in levels of sodium, potassium, and chloride did not occur in relation to administration before and after the administration. Phosphorus is an important electrolyte whose level increase due to an excretory disorder and renal hyperparathyroidism when 85% of a renal function is lost. Since administration of an excessive amount of cardiac drug reduces a renal function, phosphorus level monitoring is very important in dogs to which cardiac drugs are administered. In the present experiment, a change in phosphorus level was not observed before and after administration, and dogs with clinically significant hyperphosphatemia were not observed throughout the administration periods.

### 5. Biomarker index test

As a biomarker index test of Example 1, changes in kidney marker (SDMA), pancreatitis marker (cPL), and heart failure marker (NT-proBNP) were tested. Main biomarker test findings of dogs with heart failure due to pulmonary hypertension are an increase in the kidney marker (SDMA), an increase in the pancreatitis marker (cPL), and an increase in the heart failure marker (NT-proBNP).

SDMA is an abbreviation for symmetric dimethylarginine, and is a renal failure prognosis evaluation marker currently used most widely in veterinary clinical practice. Dogs with stage 1 renal failure are classified as SDMA of 18 or lower, dogs with stage 2 renal failure are classified as SDMA of 18 to 35, dogs with stage 3 renal failure are classified as SDMA of 36 to 54, and dogs with terminal renal failure are classified as SDMA of 54 or higher.

cPL is an abbreviation for canine pancreatic lipase, and is a prognostic evaluation marker of a dog with pancreatitis currently used most widely in veterinary clinical practice. cPL of 200 or lower indicates normal, cPL of 200 to 400 indicates borderline pancreatitis, and cPL of 400 or higher indicates pancreatitis.

NT-proBNP is an abbreviation for N-terminal pro-B-type natriuretic peptide, and is a prognostic evaluation marker of a dog with heart failure currently used most widely in veterinary clinical practice. NT-proBNP of 900 or lower indicates normal, NT-proBNP of 900 to 1800 indicates borderline heart failure, and NT-proBNP of 400 or higher indicates heart failure.

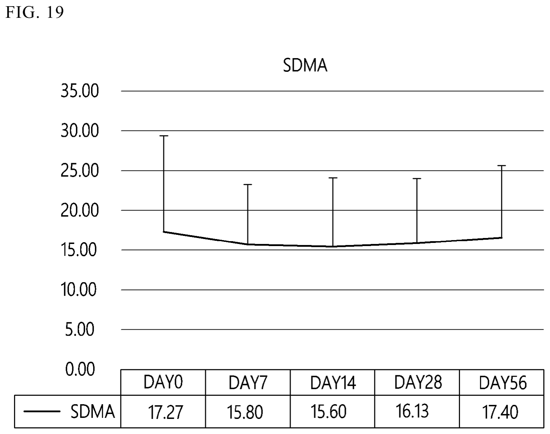

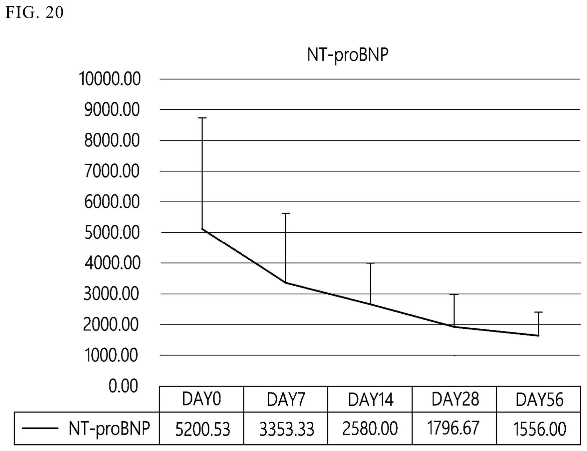

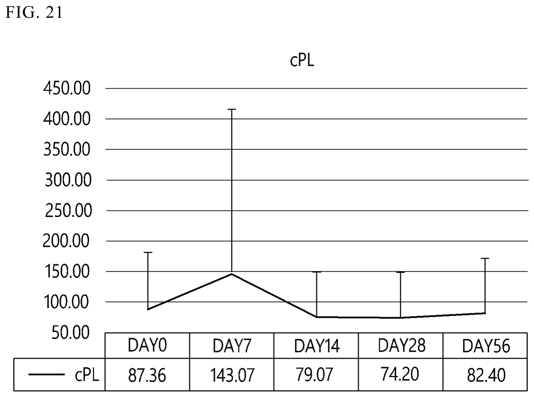

In the present evaluation, changes in biomarker indices were tested, and the results were illustrated in Table 6 and FIGS. 19 to 21.

**[Table 6]**

| Test index /Test point in time | DAY 0 | DAY 7 | DAY 14 | DAY 28 | DAY 56 |
|---|---|---|---|---|---|
| SDMA | 17.27 | 15.80 | 15.60 | 16.13 | 17.40 |
| NT-proBNP | 5200.53 | 3353.33 | 2580.00 | 1796.67 | 1556.00 |
| cPL | 87.36 | 143.07 | 79.07 | 74.20 | 82.40 |

FIGS. 19 to 21 are graphs illustrating results of a biomarker test of Example 1. FIGS. 19 to 21 illustrate changes in SDMA, NT-proBNP, and cPL as biomarkers, respectively.

Referring to Table 6 and FIGS. 19 to 21, NT-proBNP, which is the heart failure marker, gradually decreased after administration and showed a gradual decrease as a administration period passed. In particular, an average concentration of NT-proBNP after 56 days of administration decreased to 1/3 of a level before administration. SDMA, which is the kidney marker, and cPL, which is the pancreatic marker, did not show great changes before and after administration. Since dogs with systemic diseases were excluded during a selection process of target sick dogs participating in the test, it seems that kidney marker and pancreatic marker values did not change significantly before and after administration.

### 6. Chest radiograph evaluation

As a chest radiograph evaluation of Example 1, VHS, which is an evaluation index for a heart size, VLAS, which is an evaluation index for a left atrial size, and a lung field pattern were evaluated.

VHS, which is the evaluation index for a heart size, is an abbreviation for vertebral heart scale, and is a method of quantitatively evaluating a heart size on radiograph.

VLAS, which is the index for evaluating a left atrial size, is an abbreviation for vertebral left atrial score, and is a method of evaluating a heart size on a chest radiograph.

The lung field pattern is a method of evaluating how clearly lung lobes are visible on radiograph, and is mainly used to confirm whether the lungs are filled with water or inflamed.

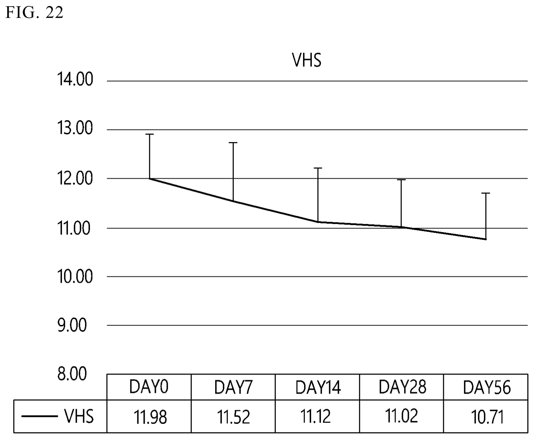

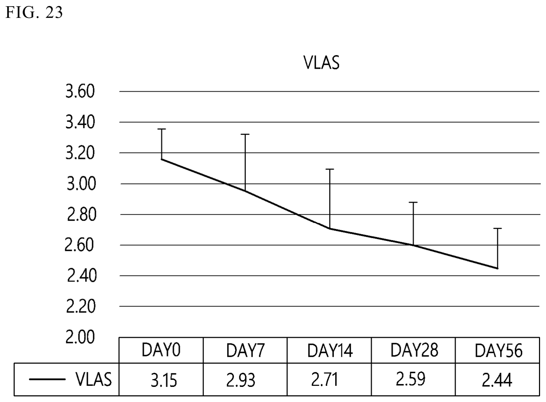

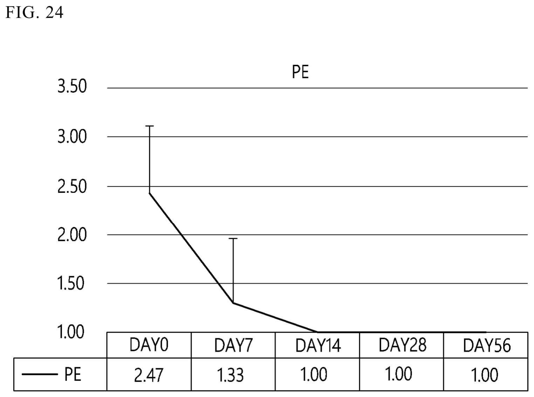

Results of the chest radiograph evaluation were illustrated in Table 7 and FIGS. 22 to 24.

**[Table 7]**

| Test index/Test point in time | DAY 0 | DAY 7 | DAY 14 | DAY 28 | DAY 56 |
|---|---|---|---|---|---|
| VHS | 11.98 | 11.52 | 11.12 | 11.02 | 10.71 |
| VLAS | 3.15 | 2.93 | 2.71 | 2.59 | 2.44 |
| lung field pattern | 2.47 | 1.33 | 1.00 | 1.00 | 1.00 |

FIGS. 22 to 24 are graphs illustrating results of a chest radiograph evaluation of Example 1. FIGS. 22 to 24 illustrate evaluation results of VHS, VLAS, and a lung field pattern as the results of the chest radiograph evaluation, respectively.

Referring to Table 7 and FIGS. 22 to 24, VHS, which is the evaluation index for a heart size, started to decrease from 7 days of administration (DAY 7) and continuously decreased until 56 days of administration (DAY 56), and VLAS, which is the index for a left atrial size, also started to decrease from 7 days of administration (DAY 7) and continuously decreased until 56 days of administration (DAY 56). Infiltration of a lung field also improved to a normal lung lobe pattern in most sick dogs at a test of 7 days of administration (DAY 7), and sick dogs with infiltration of a lung field were no longer observed at a test point in time of 14 days of administration (DAY 14).

### 7. Cardiac ultrasound evaluation

As a cardiac ultrasound evaluation of Example 1, LA/Ao, LVIDDn, MVE, and MV E/E' were tested using cardiac ultrasound to evaluate a function of the left cardiac system. In addition, TR velocity, PR velocity, RVOT ET/AT, and RVOT AT were tested using cardiac ultrasound to evaluate a function of the right cardiac system.

LA/Ao is an abbreviation for an aorta to left atria diameter ratio, and an index that evaluates a degree of left atrial enlargement. Dogs with ISACHC I heart failure show a LA/Ao value of 1.4 to 1.8, dogs with ISACHC II heart failure show a LA/Ao value of 1.8 to 2.0, and dogs with ISACHC III heart failure show a LA/Ao value of 2.0 or higher. Normal LA/Ao is 1.2 to 1.4, and LA/Ao of 1.2 or lower is LA/Ao of normal LA/Ao or lower and is mainly caused by dehydration.

LVIDdN is an index that evaluates an end-diastolic diameter of the left ventricle regardless of a sick dog's weight, and when LVIDdN is 20 or higher, it is usually considered that the end-diastolic diameter of the left ventricle increases.

MVE indicates a blood flow velocity of a bicuspid valve during filling, and mainly increases as heart failure becomes more severe. Dogs with ISACHC I heart failure show MVE of 0.8 to 1.0 m/s, dogs with ISACHC II heart failure MVE of 1.0 to 1.2 m/s, and dogs with ISACHC III heart failure show MVE of 1.2 m/s or higher. Normal MVE is 0.8 m/s or lower.

MV E/E' is a ratio between a blood flow velocity of the bicuspid valve and a tissue Doppler velocity of the bicuspid valve annulus, and is an index that may predict a pressure in the left atrial. When MV E/E' is 9 or higher, the left atrial pressure is 20 mmHg, and when MV E/E' is 12 or higher, the left atrial pressure is 30 mmHg or more. Normally, the left atrial pressure should not exceed 20 mmHg. An increase in value means an increase in the left atrial pressure.

The TR velocity is a regurgitation velocity of a tricuspid value blood flow (in a dog with pulmonary hypertension, a tricuspid valve blood flows in an opposite direction to an original direction during systole due to an increase in resistance in the right cardiac system and pulmonary blood vessels). The faster the regurgitation velocity of the blood flow, the more severe the pulmonary hypertension. When the TR velocity is 2.8 m/s or higher, it is diagnosed as pulmonary hypertension.

The PR velocity is a regurgitation velocity of a pulmonary artery blood flow (in a dog with pulmonary hypertension, a pulmonary artery blood flows in an opposite direction to an original direction during systole due to an increase in resistance in the right cardiac system and pulmonary blood vessels). The faster the regurgitation velocity of the blood flow, the more severe the pulmonary hypertension. When the PR velocity is 2.2 m/s or higher, it is diagnosed as pulmonary hypertension.

In a measurement of pulmonary artery blood flow, RVOT ET/AT is mainly composed of an acceleration time (AT), an ejection time (ET), and a deceleration time (DT). When the resistance of the pulmonary blood vessels increases, the acceleration time becomes shorter and the ejection time becomes longer. AT/ET is an echocardiographic index that may evaluate how severe pulmonary hypertension is by comparing the acceleration time and the ejection time with each other. Normal AT/ET is 0.42 or higher, and AT/ET significantly decreases to 0.30 or lower in dogs with severe pulmonary hypertension. In other words, the lower the AT/ET, the more severe the pulmonary hypertension.

In a measurement of pulmonary artery blood flow, RVOT AT is mainly composed of an acceleration time (AT), an ejection time (ET), and a deceleration time (DT). When the resistance of the pulmonary blood vessels increases, the acceleration time becomes shorter. This index is an echocardiographic index that may evaluate how severe pulmonary hypertension is. The lower the RVOT AT, the shorter the acceleration time, which means a severe pulmonary hypertension status.

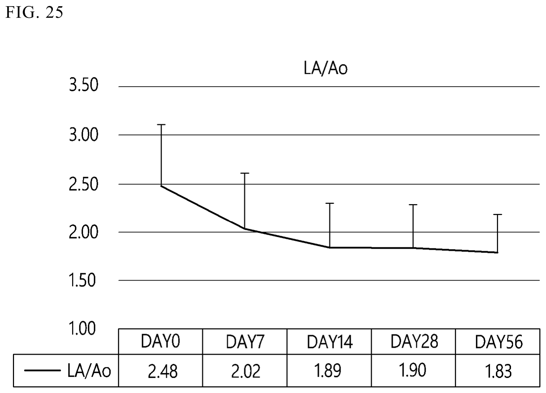

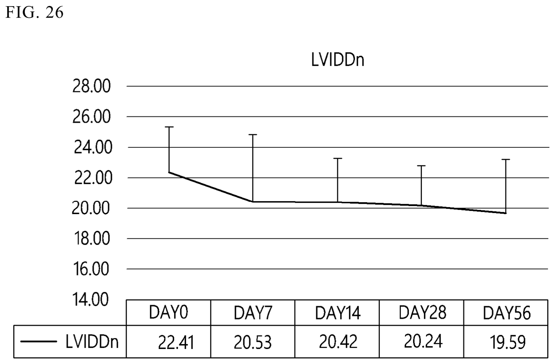

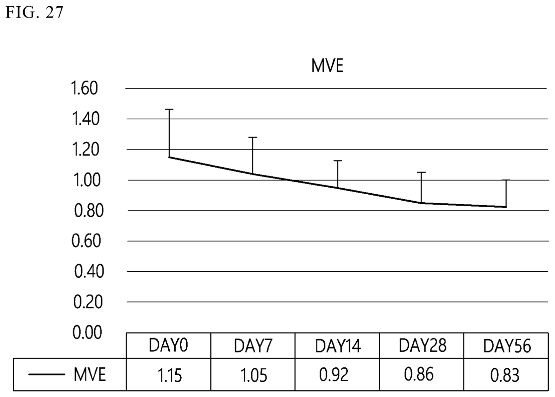

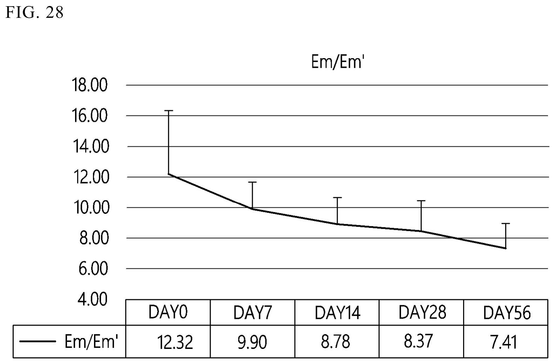

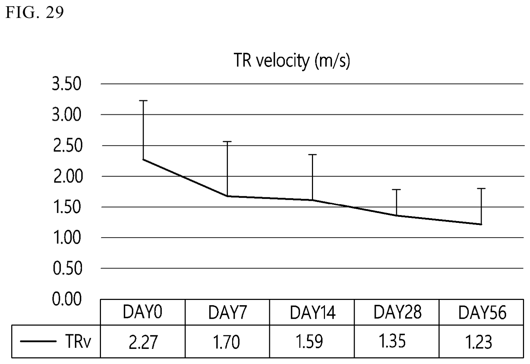

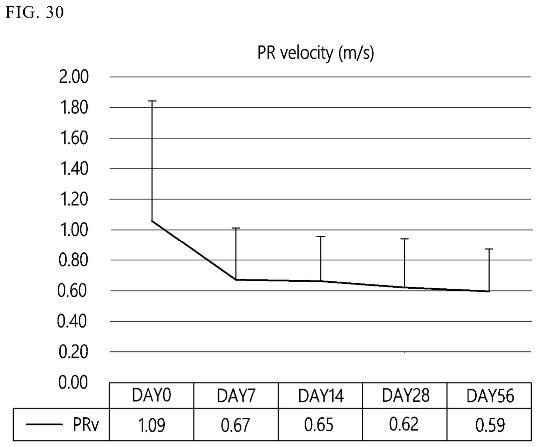

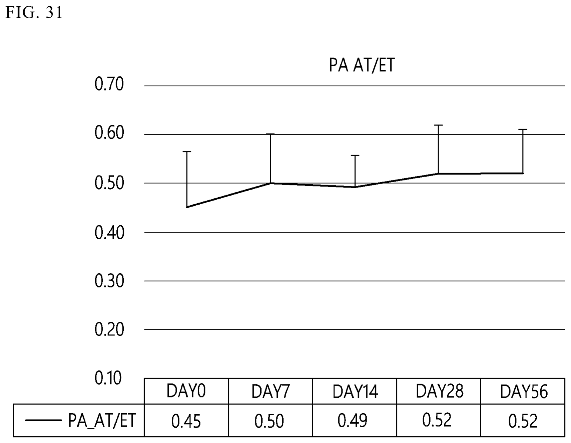

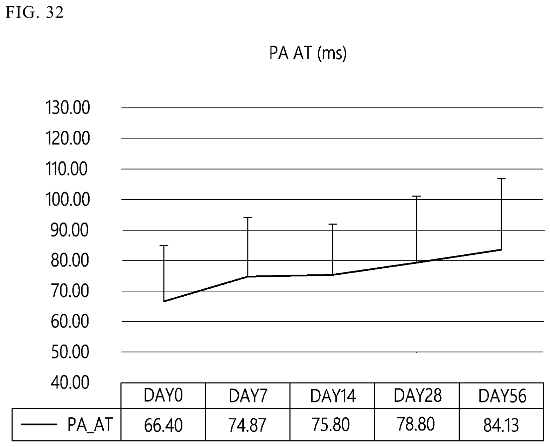

The results of the present cardiac ultrasound evaluation were illustrated in Table 8 and FIGS. 25 to 32.

**[Table 8]**

| Test index /Test point in time | DAY 0 | DAY 7 | DAY 14 | DAY 28 | DAY 56 |
|---|---|---|---|---|---|
| LA/Ao | 2.48 | 2.02 | 1.89 | 1.90 | 1.83 |
| LVIDdN | 22.41 | 20.53 | 20.42 | 20.24 | 19.59 |
| MVE | 1.15 | 1.05 | 0.95 | 0.86 | 0.83 |
| MV E/E' | 12.32 | 9.90 | 8.78 | 8.37 | 7.41 |
| TR velocity | 0.45 | 0.50 | 0.49 | 0.52 | 0.52 |
| PR velocity | 66.40 | 74.87 | 75.80 | 78.80 | 84.13 |
| RVOT ET/AT | 2.27 | 1.70 | 1.59 | 1.35 | 1.23 |
| RVOT AT | 1.09 | 0.67 | 0.65 | 0.62 | 0.59 |

FIGS. 25 to 32 are graphs illustrating results of a cardiac ultrasound evaluation of Example 1. FIGS. 25 to 32 illustrate evaluation results of LA/Ao, LVIDDn, MVE, MV E/E', TR velocity, PR velocity, RVOT ET/AT, and RVOT AT as the results of the cardiac ultrasound evaluation, respectively. Referring to Table 8 and FIGS. 25 to 32, also in the present experiment, it could be confirmed that such left cardiac system indices were significantly improved in an evaluation after 7 days of administration (DAY 7), and such an improvement effect continued until 56 days of administration (DAY 56). Since the circulatory systems of dogs and cats are closed circulatory systems, disease occurring on the left or right side of the heart ultimately transfers a pressure to an opposite side of the heart. Accordingly, in many dogs with left heart failure, right heart failure occurs over time, and a main cause of such a phenomenon is pulmonary hypertension. Also in the present experiment, pulmonary hypertension was observed in many sick dog groups, and it could confirmed that test indices of the pulmonary hypertension were significantly improved when test drug administration was started in these sick dogs. Furthermore, it could be confirmed that such an improvement effect continued until 56 days (DAY 56) of administration.

### 8. Heart failure index evaluation

As a heart failure index evaluation of Example 1, an ISACHC stage evaluation was performed. In an ISACHC stage evaluation method, a classification index for dogs with heart failure proposed by the International Small Animal Cardiac Heart Council is divided into a total of 5 stages: 1. ISACHC IA asymptomatic sick dog group without cardiomegaly, 2. ISACHC IB asymptomatic sick dog group with cardiomegaly, 3. ISACHC II sick dog group with mild-moderate heart failure, 4. ISACHC IIIA dog group with severe heart failure in which pulmonary edema occurs, 5. ISACHC IIIB dog group with end-stage heart failure having recurrent pulmonary edema.

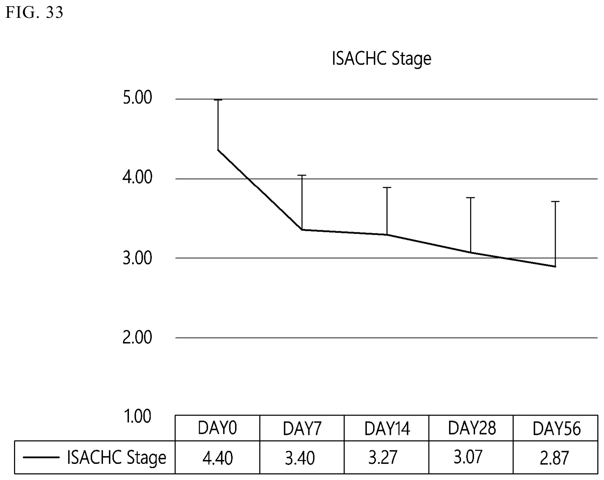

The results of the ISACHC stage evaluation were illustrated in Table 9 and FIG. 33.

**[Table 9]**

| Test index /Test point in time | DAY 0 | DAY 7 | DAY 14 | DAY 28 | DAY 56 |
|---|---|---|---|---|---|
| ISACHC stage | 4.40 | 3.40 | 3.27 | 3.07 | 2.87 |

FIG. 33 is a graph illustrating an evaluation result of an ISACHC stage of Example 1.

Referring to Table 9 and FIG. 33, in the present test, most of the sick dogs were in ISACHC IIIA and IIIB statuses, but a significant improved status was confirmed from 7 days of administration (DAY 7), and such an improved status continued until 56 days of administration (DAY 56).

### 9. Conclusion according to experimental results

As a result of the evaluation of Example 1, it could be seen that the composition for preventing and treating heart disease according to an embodiment is effective in preventing and treating heart disease in animals such as dogs and cats, by including pimobendan, which is a cardiotonic ingredient, enalapril, which is a vasodilator, and torsemide and spironolactone, which are diuretic ingredients.

The composition for preventing and treating heart disease of animals according to an embodiment of the present disclosure showed an improvement in heart function in clinical indices for heart failure as the result of the clinical test, and showed an effect in treating heart failure. In particular, it could be seen that the composition according to the present disclosure also improved pulmonary hypertension, which is a complication due to heart failure. Side effects related to the kidney, the liver, and the electrolyte related to drug administration were not observed within the administration periods. It could also be confirmed that a heart failure stage was gradually and continuously improved after administration.

### <Example 2> Preparation of composition for preventing and treating heart disease

In order to more specifically confirm efficacy of the composition of Example 1 on the pulmonary hypertension, various Comparative Examples were prepared and evaluations similar to those described above were performed.

Preparation Example 1, which is a composition for treating heart disease, was prepared by mixing compositions in Table 10 with each other. A unit in Table 10 is mg. A tablet having a content of 100 mg per tablet was prepared by tableting a composition having the following composition.

In addition, Comparative Examples 1 to 3, which are compositions for treating heart disease, were prepared by mixing compositions in Table 11 with each other. A unit in Table 11 is mg. A tablet having a content of 100 mg per tablet was prepared by tableting a composition having the following composition.

**[Table 10]**

| Ingredient | Content (100 mg) |
|---|---|
| Pimobendan | 0.5mg |
| Enalapril | 1mg |
| Torsemide | 0.2mg |
| Spironolactone | 2mg |
| Preservative | Appropriate amount |
| Other additive | Appropriate amount |

**[Table 11]**

| Ingredient | Comparative Example 1 | Comparative Example 2 | Comparative Example 3 |
|---|---|---|---|
| | Content (100 mg) | Content (100 mg) | Content (100 mg) |
| Pimobendan | 0.5mg | 0.5mg | 0.5mg |
| Enalapril | - | 1mg | 1mg |
| Torsemide | - | - | 0.2mg |
| Spironolactone | - | - | - |
| Preservative | Appropriate | Appropriate | Appropriate |
| | amount | amount | amount |
| Other additive | Appropriate amount | Appropriate amount | Appropriate amount |

### <Experimental Example 2> Confirmation of heart disease prevention and treatment effects

The following experiment was performed in order to confirm heart disease prevention and treatment effects by the composition of Preparation Example 1 and the compositions of Comparative Examples 1 to 3.

### <Experimental Method>

A test group to which the composition of Preparation Example 1 and the compositions of Comparative Examples 1 to 3 are to be administered and a placebo group to which a placebo is to be administered were selected, and an experiment was performed to evaluate the efficacy and stability according to administration. As the test group and the placebo group, 40 dogs, which are experimental animals diagnosed with heart failure of an ISACHC stage III or higher, were selected. The 40 experimental animals were selected as the same gender compositions of small dog breeds and 20 females and 20 males.

Heart failure statuses of the selected experimental animals were classified according to direct examination by a veterinary cardiologist through a clinical test, a chest radiograph test, and a cardiac ultrasound test, and an ACVIM pulmonary hypertension guideline. Dogs with other organ diseases in the clinical test were excluded.

As a test drug administration method, the tablet of Preparation Example 1 prepared in Example 2 was orally administered to the experimental animal twice a day, and 1/2 tablet per weight of 1 kg of the experimental animal was administered when it was administered once. The tablet was administered at 12-hour intervals in the morning and evening at the same time regardless of meals, and an administration period was 30 days.

The tablets of Comparative Examples 1 to 3 prepared in Example 2 were also orally administered to the experimental animal twice a day. However, one tablet of Comparative Example 1 per weight of 5 kg of the experimental animal was administered to the experimental animal, and 1/2 tablets of Comparative Examples 2 and 3 per weight of 1 kg the experimental animal was administered to the experimental animal. The tablet was administered at 12-hour intervals in the morning and evening at the same time regardless of meals, and an administration period was 30 days.

In a plan of the present experiment, an administration experiment was performed on dogs with heart failure caused by various causes including valvular disease. ISACHC stage III heart failure is a severe heart failure status, a group of dogs with the ISACHC stage III heart failure is a group with obvious clinical symptoms of heart disease such as pulmonary edema or faint, and pulmonary hypertension indices of dogs with pulmonary hypertension as complications of heart disease among such dogs were separately recorded, and improvement effects according to drug administration were also investigated. When the pulmonary edema or the clinical symptoms worsened within the administration period, the administration was stopped, a rescue drug (a diuretic or a cardiotonic) was administered instead, and only indices of dogs whose administrations were not changed until an end of the experiment were evaluated and average values of the indices were compared with each other.

Test intervals for drug administration were a day before administration (DAY 0) and 30 days of administration (DAY 30).

Specific observation items and test method are as follows.

### Observation items

1) Evaluation of heart and lung field on radiograph: VHS, VLAS, lung field pattern
2) Cardiac ultrasound: LA/Ao, LVIDdn, MVE, E/E', TR velocity, PR velocity, RVOT ET/AT, RVOT AT measurement
3) Pulmonary hypertension evaluation: None (0), mild (1), moderate (2), severe (3)
4) Heart failure stage: ISACHC lA(1), ISACHC 1B(2), ISACHC 2(3), ISACHC 3A(4), ISACHC 3B(5)
5) As clinical test items, exercise intolerance (1 to 4; 1 is best and 4 is worst), appetite (1 to 4), respiratory effort (1 to 4), cough (1 to 4), and faint (1 to 4) were checked with different numerical values, respectively.

As the observation test method, tests for clinical test items were performed on a day before administration (DAY 0) and 30 days of administration (DAY 30), respectively, and test results were recorded by a veterinarian and guardian, respectively Tests for radiograph, cardiac ultrasound, and biomarker, which are the observation items, were performed at designated hospitals and test results were recorded.

Effect evaluation criteria according to test results for the respective clinical test items were as follows.

### 1) Effect evaluation criteria

- Whether or not clinical symptoms are improved: increase in vitality , decrease in the number of times of cough, increase in appetite, decrease in respiratory effort, decrease in the number of times of faint
- Improvement of heart size and lung field infiltration status on chest radiograph
- Improvement of cardiac test indices on cardiac ultrasound

### 2) Effect evaluation method

- Whether or not clinical symptoms are improved: evaluated through guardian records and veterinarian examination records
- Improvement of CBC and serum biochemical indices: evaluated for liver, kidney, pancreas, and electrolyte test items
- Relief of pulmonary hypertension on chest radiograph: determined through evaluation of VHS (heart size), VLAS (left atrial size), and lung field pattern (lung field infiltration findings)
- Improvement of cardiac test indices on cardiac ultrasound: LA/Ao (aorta to left atria diameter ratio), LVIDdn (left ventricular end-diastolic diameter), MVE (bicuspid valve filling velocity), E/E' (bicuspid valve filling velocity to tissue Doppler velocity ratio)
- Decrease in stage of pulmonary hypertension: determined through evaluation of TR velocity (tricuspid value regurgitation velocity), PR velocity (pulmonary artery regurgitation velocity), RVOT ET/AT (right ventricular outflow tract ejection time/acceleration time), and RVOT AT (right ventricular outflow tract acceleration time)
- Improvement of heart failure stage: decrease in heart failure stage classified by the International Small Animal Cardiac Heart Council (ISACHC) classification method

### 3) Statistical interpretation method

- Normal distribution of each test index was tested through the Kolmogorov-Smirnov test.
- A difference in test indices between respective groups was compared through the Mann-Whitney U test, and the Wilcoxson signed rank test was performed for a comparison before and after administration.
- P < 0.05 was evaluated as being statistically significant.

### <Experimental Result 2>

### 1. Clinical test

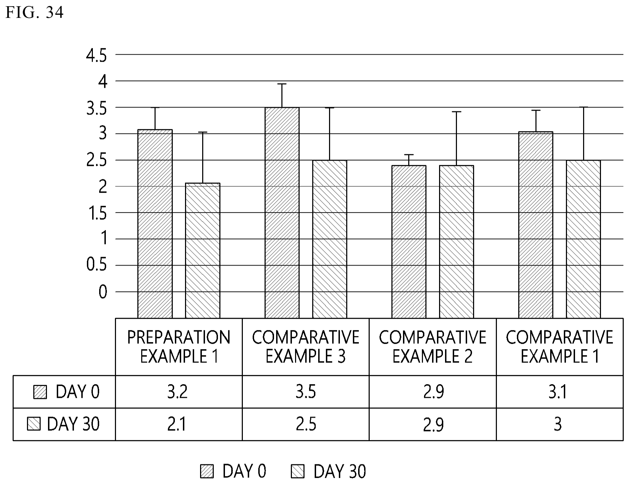

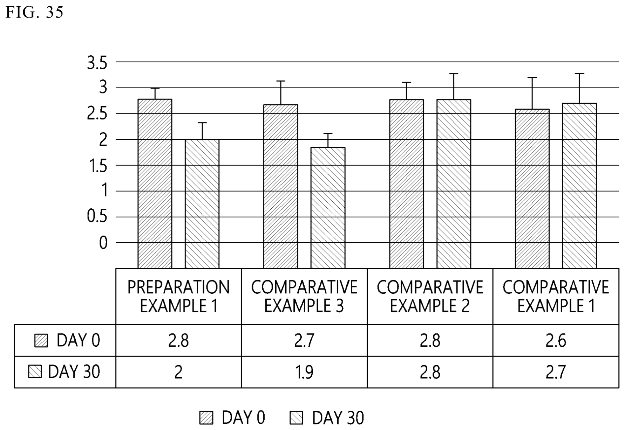

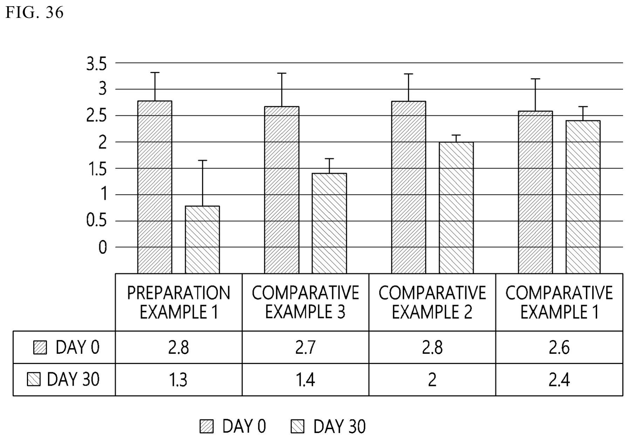

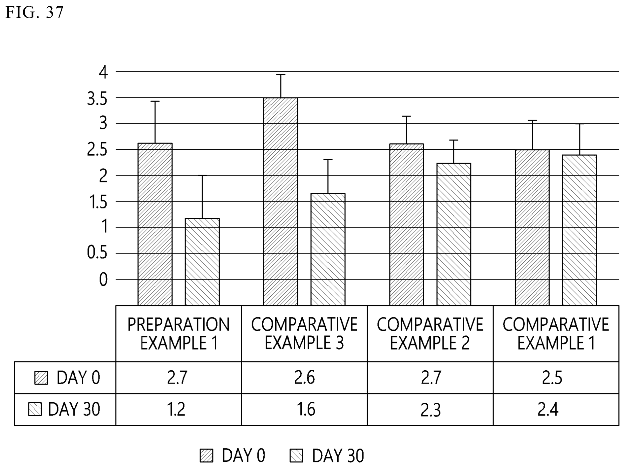

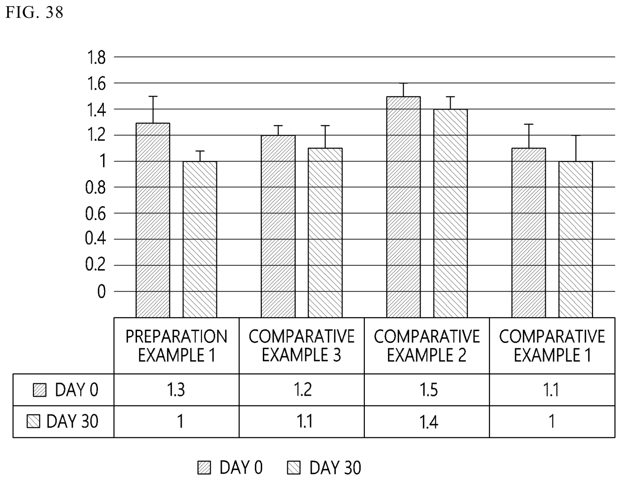

As results of a clinical test of Example 2, exercise intolerance, appetite, faint, respiratory effort, and cough evaluation results were illustrated in Table 12 and FIGS. 34 to 38.

**[Table 12]**

| Test index-Test point in time/Test target | | Preparation Example 1 | Comparative Example 1 | Comparative Example 2 | Comparative Example 3 |
|---|---|---|---|---|---|
| Exercise intolerance (1 to 4)* | DAY0 | 3.2 | 3.1 | 2.9 | 3.5 |
| | DAY30 | 2.1 | 3 | 2.9 | 2.5 |
| Appetite (1 to 4) | DAY0 | 2.8 | 2.6 | 2.8 | 2.7 |
| | DAY30 | 2 | 2.7 | 2.8 | 1.9 |
| Faint (1 to 4) | DAY0 | 1.3 | 1.1 | 1.5 | 1.2 |
| | DAY30 | 1 | 1 | 1.4 | 1.1 |
| Respiratory effort (1 to 4) | DAY0 | 2.8 | 2.6 | 2.8 | 2.7 |
| | DAY30 | 1.3 | 2.4 | 2 | 1.4 |
| Cough (1 to 4) | DAY0 | 2.7 | 2.5 | 2.7 | 2.6 |
| | DAY30 | 1.2 | 2.4 | 2.3 | 1.6 |

In Table 12, numerical values 1 to 4 of clinical evaluation results were evaluated as indicating the following cases, respectively.
- Exercise intolerance: 1. None at all, 2. Feels tired after a walk for a long time, 3. Has difficulty in walking during a walk, 4. Refuses to walk
- Appetite: 1. Very good, 2. Good, 3. Picky eater (Tries to eat only delicious food), 4. Refuses to eat food
- Respiratory effort: 1. None at all, 2. Hyperventilates after having fun, 3. Hyperventilates within 30 minutes even during a rest, 4. Hyperventilates even during sleep
- Cough: 1. None at all, 2. Coughs when excited or drinking water, 3. Coughs less than 10 times a day regardless of excitement or drinking water, 4. Cannot sleep because of cough
- Faint: 1. None at all, 2. Faints once or less a day after exercise or excitement, 3. Faints once or less a day regardless of exercise or excitement, 4. Faints once or more a day regardless of exercise or excitement

FIGS. 34 to 38 are graphs illustrating results of a clinical test of Example 2. FIGS. 34 to 38 illustrate exercise intolerance, appetite, respiratory effort, cough, and faint evaluation results of each of the test group and the placebo group, respectively, as the results of the clinical test.

Referring to FIGS. 34 to 38, in the case of an exercise intolerance evaluation, a symptom of exercise intolerance was significantly improved in a group to which the composition of Preparation Example 1 or Comparative Example 3 is administered at an end of the test (DAY 30), whereas symptoms of exercise intolerance were not significantly improved in groups to which the compositions of Comparative Example 1 and Comparative Example 2 are administered. Such a symptom improvement effect was most clearly observed in the group to which the composition of Preparation Example 1 is administered.

In the case of an appetite evaluation, an appetite was significantly improved in the group to which the composition of Preparation Example 1 or Comparative Example 3 is administered at an end of the test (DAY 30), whereas appetites were not significantly improved in the groups to which the compositions of Comparative Example 1 and Comparative Example 2 are administered. Such a symptom improvement effect was most clearly observed in the group to which the composition of Preparation Example 1 is administered.

In the case of a respiratory effort evaluation, an effortful respiratory symptom was significantly improved in the group to which the composition of Preparation Example 1 or Comparative Example 3 is administered at an end of the test (DAY 30), whereas a partial improvement effect of an effortful respiratory symptom was observed in the group to which the composition of Comparative Example 2 is administered and an effortful respiratory symptom was not significantly improved in the group to which the composition of Comparative Example 1 is administered. Such symptom improvement effects were most clearly observed in the groups to which the compositions of Preparation Example 1 and Comparative Example 3 are administered, and were similarly observed in the groups to which the compositions of Preparation Example 1 and Comparative Example 3 are administered.

In the case of a cough evaluation, a cough symptom was significantly improved in the group to which the composition of Preparation Example 1 or Comparative Example 3 is administered at an end of the test (DAY 30), whereas a partial improvement effect of a cough was observed in the group to which the composition of Comparative Example 2 is administered and a cough symptom was not significantly improved in the group to which the composition of Comparative Example 1 is administered. Such a symptom improvement effect was most clearly observed in the group to which the composition of Preparation Example 1 is administered.

In the case of a faint evaluation, an improvement effect of a faint symptom was observed in most groups to which drugs are administered, but was most clearly observed in the group to which the composition of Preparation Example 1 is administered.

As described above, it could be seen from results of 1. Clinical test that the composition of Preparation Example 1 showed an improved effect in evaluations of exercise intolerance, appetite, respiratory effort, cough, faint, and the like, compared to the compositions of Comparative Examples 1 to 3.

### 2. Chest radiograph evaluation

As a chest radiograph evaluation of Example 2, VHS, which is an evaluation index for a heart size, VLAS, which is an evaluation index for a left atrial size, and a lung field pattern were evaluated.

VHS, which is the evaluation index for a heart size, is an abbreviation for vertebral heart scale, and is a method of quantitatively evaluating a heart size on radiograph.

VLAS, which is the index for evaluating a left atrial size, is an abbreviation for vertebral left atrial score, and is a method of evaluating a heart size on a chest radiograph.

The lung field pattern is a method of evaluating how clearly lung lobes are visible on radiograph, and is mainly used to confirm whether the lungs are filled with water or inflamed.

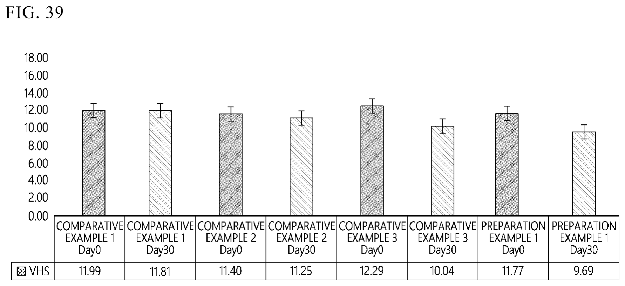

Results of the chest radiograph evaluation were illustrated in Table 13 and FIGS. 39 to 41.

**[Table 13]**

| Test index-Test point in time/Test target | Preparation Example 1 | Comparative Example 1 | Comparative Example 2 | Comparative Example 3 |
|---|---|---|---|---|
| VHS | DAY0 | 11.77 | 11.99 | 11.40 |
| | DAY30 | 9.69 | 11.81 | 11.25 |
| VLAS | DAY0 | 3.27 | 3.09 | 3.18 |
| | DAY30 | 2.63 | 2.95 | 3.03 |
| Lung field pattern | DAY0 | 2.60 | 2.30 | 2.56 |
| | DAY30 | 1.00 | 2.50 | 2.42 |

FIGS. 39 to 41 are graphs illustrating results of a chest radiograph evaluation of Example 2. FIGS. 39 to 41 illustrate evaluation results of VHS, VLAS, and a lung field pattern (PE) as the results of the chest radiograph evaluation, respectively.

As heart failure progresses, the heart becomes greater, and the left atrium expands. When the left atrium becomes too great, the pulmonary blood vessels expand, such that water infiltrates into the lung field, resulting in pulmonary edema, which in turn causes a cough or an effortful respiratory symptom.

The evaluation result of the VHS of FIG. 39 may be a result of quantitatively evaluating a heart size on radiograph. Referring to Table 13 and FIG. 39, in relation to the VHS, which is an evaluation index for the heart size, a significant change in heart size was not observed in the groups to which the compositions of Comparative Example 1 and Comparative Example 2 are administered at an end of the test (DAY 30), whereas an obvious decrease in heart size was observed in the groups to which the compositions of Preparation Example 1 and Comparative Example 3 are administered at an end of the test (DAY 30) compared to that before administration.

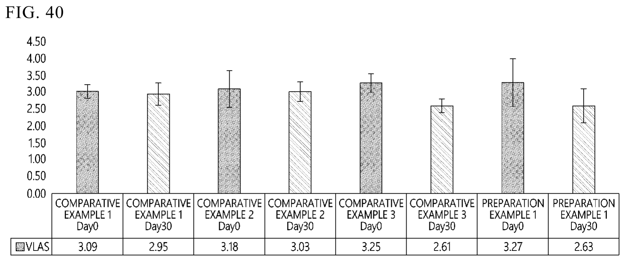

The evaluation result of the VLAS of FIG. 40 may be a result of evaluating a left atrial size on a chest radiograph. Referring to Table 13 and FIG. 40, in relation to the VLAS, which is an evaluation index for the left atrial size, a significant change in left atrial size was not observed in the groups to which the compositions of Comparative Example 1 and Comparative Example 2 are administered at an end of the test (DAY 30), whereas an obvious decrease in left atrial size was observed in the groups to which the compositions of Preparation Example 1 and Comparative Example 3 are administered at an end of the test (DAY 30) compared to that before administration.

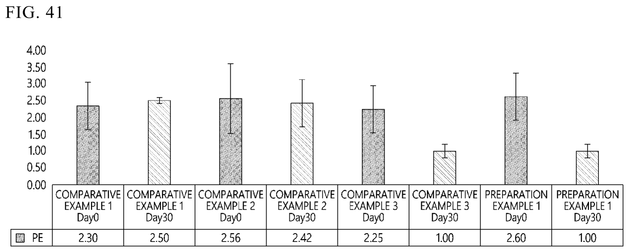

The evaluation result of the lung field pattern (PE) of FIG. 41 is a method of evaluating how clearly the lung lobes are visible on radiograph, and is mainly used to confirm whether the lungs are filled with water or inflamed. Referring to Table 13 and FIG. 41, lung field pattern (PE) findings were not significantly improved in the groups to which the compositions of Comparative Example 1 and Comparative Example 2 are administered at an end of the test (DAY 30). In particular, it could be seen that the lung field pattern (PE) in the group to which the composition of Comparative Example 1 is administered was more severe than that before the administration. Lung field pattern (PE) findings were obviously improved in the groups to which the compositions of Preparation Example 1 and Comparative Example 3 are administered at an end of the test (DAY 30) compared to those before administration.

### 3. Cardiac ultrasound evaluation

As a cardiac ultrasound evaluation of Example 2, LA/Ao, LVIDDn, MVE, and MV E/E' were tested using cardiac ultrasound to evaluate a function of the left cardiac system. In addition, TR velocity, PR velocity, RVOT ET/AT, and RVOT AT were tested using cardiac ultrasound to evaluate a function of the right cardiac system.

LA/Ao is an abbreviation for an aorta to left atria diameter ratio, and an index that evaluates a degree of left atrial enlargement. Dogs with ISACHC I heart failure show a LA/Ao value of 1.4 to 1.8, dogs with ISACHC II heart failure show a LA/Ao value of 1.8 to 2.0, and dogs with ISACHC III heart failure show a LA/Ao value of 2.0 or higher. Normal LA/Ao is 1.2 to 1.4, and LA/Ao of 1.2 or lower is LA/Ao of normal LA/Ao or lower and is mainly caused by dehydration.

LVIDdN is an index that evaluates an end-diastolic diameter of the left ventricle regardless of a sick dog's weight, and when LVIDdN is 20 or higher, it is usually considered that the end-diastolic diameter of the left ventricle increases.

MVE indicates a blood flow velocity of a bicuspid valve during filling, and mainly increases as heart failure becomes more severe. Dogs with ISACHC I heart failure show MVE of 0.8 to 1.0 m/s, dogs with ISACHC II heart failure MVE of 1.0 to 1.2 m/s, and dogs with ISACHC III heart failure show MVE of 1.2 m/s or higher. Normal MVE is 0.8 m/s or lower.

MV E/E' is a ratio between a blood flow velocity of the bicuspid valve and a tissue Doppler velocity of the bicuspid valve annulus, and is an index that may predict a pressure in the left atrial. When MV E/E' is 9 or higher, the left atrial pressure is 20 mmHg, and when MV E/E' is 12 or higher, the left atrial pressure is 30 mmHg or more. Normally, the left atrial pressure should not exceed 20 mmHg. An increase in value means an increase in the left atrial pressure.

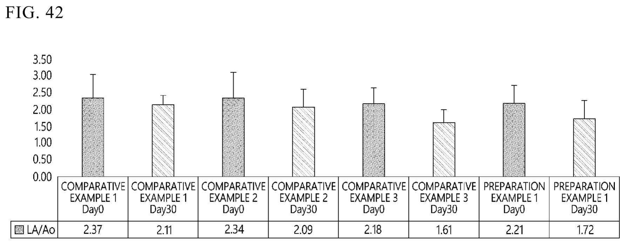

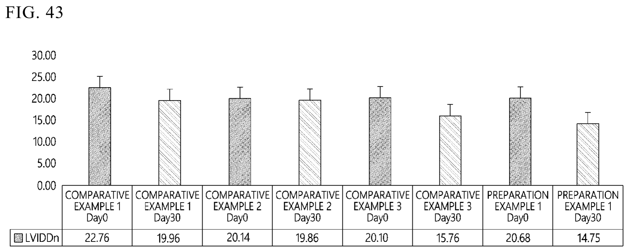

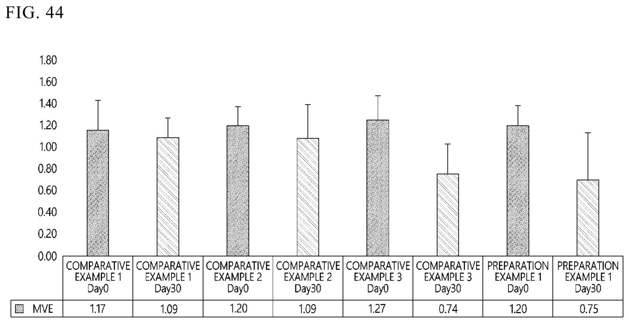

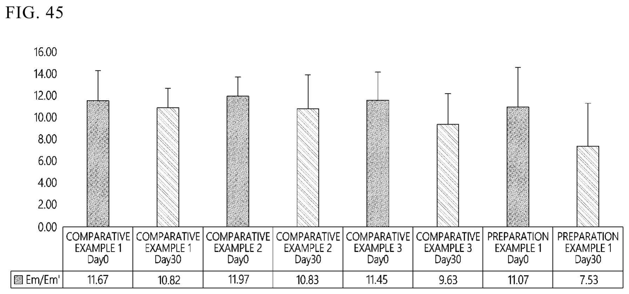

The results of the cardiac ultrasound evaluation were illustrated in Table 14 and FIGS. 42 to 45.

**[Table 14]**

| Test index-Test point in time/Test target | Preparation Example 1 | Comparative Example 1 | Comparative Example 2 | Comparative Example 3 |
|---|---|---|---|---|
| LA/Ao | DAY0 | 2.21 | 2.37 | 2.34 |
| | DAY30 | 1.72 | 2.11 | 2.09 |
| LVIDdN | DAY0 | 20.68 | 22.76 | 20.14 |
| | DAY30 | 14.75 | 19.96 | 19.86 |
| MVE | DAY0 | 1.20 | 1.17 | 1.20 |
| | DAY30 | 0.75 | 1.09 | 1.09 |
| MV E/E' | DAY0 | 11.07 | 11.67 | 11.97 |
| | DAY30 | 7.53 | 10.82 | 10.83 |

FIGS. 42 to 45 are graphs illustrating results of a cardiac ultrasound evaluation of Example 2. FIGS. 42 to 45 illustrate evaluation results of LA/Ao, LVIDDn, MVE, and MV E/E' as the results of the cardiac ultrasound evaluation, respectively. As heart failure progresses, the heart becomes greater, and the left atrium expands. Accordingly, as main test indices using cardiac ultrasound, LA/Ao that evaluates a degree of left atrial enlargement, LVIDDₙ that evaluates a degree of left ventricular enlargement, and MVE and MV E/E' that evaluate an increase in left atrial pressure are used.

Referring to Table 13 and FIGS. 42 to 45, in an evaluation of LA/Ao that evaluates the degree of the left atrial enlargement, a significant change in left atrial size was not observed in the groups to which the compositions of Comparative Example 1 and Comparative Example 2 are administered at an end of the test (DAY 30), whereas an obvious decrease in left atrial size was observed in the groups to which the compositions of Preparation Example 1 and Comparative Example 3 are administered at an end of the test (DAY 30) compared to that before administration.

In an evaluation of LVIDDₙ that evaluates the degree of the left ventricular enlargement, a significant change in left ventricular size was not observed in the groups to which the compositions of Comparative Example 1 and Comparative Example 2 are administered at an end of the test (DAY 30), whereas an obvious decrease in left ventricular size was observed in the groups to which the compositions of Preparation Example 1 and Comparative Example 3 are administered at an end of the test (DAY 30) compared to that before administration.

In evaluations of MVE and MV E/E' that evaluate the increase in left atrial pressure, a significant change in bicuspid valve blood flow velocity and a significant change in left atrial pressure were not observed in the groups to which the compositions of Comparative Example 1 and Comparative Example 2 are administered at an end of the test (DAY 30), whereas obvious decreases in bicuspid valve blood flow velocity and left atrial pressure were observed in the groups to which the compositions of Preparation Example 1 and Comparative Example 3 are administered at an end of the test (DAY 30) compared to those before administration. In particular, the decrease in left atrial pressure was clearly observed in the group to which the composition of Preparation Example 1 is administered.

### 4. Pulmonary hypertension index evaluation

As pulmonary hypertension and heart failure index evaluations of Example 2, TR velocity, PR velocity, RVOT ET/AT, and RVOT AT were tested using cardiac ultrasound to evaluate a function of the right cardiac system.

The TR velocity is a regurgitation velocity of a tricuspid value blood flow (in a dog with pulmonary hypertension, a tricuspid valve blood flows in an opposite direction to an original direction during systole due to an increase in resistance in the right cardiac system and pulmonary blood vessels). The faster the regurgitation velocity of the blood flow, the more severe the pulmonary hypertension. When the PR velocity is 2.8 m/s or higher, it is diagnosed as pulmonary hypertension.

The PR velocity is a regurgitation velocity of a pulmonary artery blood flow (in a dog with pulmonary hypertension, a pulmonary artery blood flows in an opposite direction to an original direction during systole due to an increase in resistance in the right cardiac system and pulmonary blood vessels). The faster the regurgitation velocity of the blood flow, the more severe the pulmonary hypertension. When the PR velocity is 2.2 m/s or higher, it is diagnosed as pulmonary hypertension.

In a measurement of pulmonary artery blood flow, RVOT ET/AT is mainly composed of an acceleration time (AT), an ejection time (ET), and a deceleration time (DT). When the resistance of the pulmonary blood vessels increases, the acceleration time becomes shorter and the ejection time becomes longer. AT/ET is an echocardiographic index that may evaluate how severe pulmonary hypertension is by comparing the acceleration time and the ejection time with each other. Normal AT/ET is 0.42 or higher, and AT/ET significantly decreases to 0.30 or lower in dogs with severe pulmonary hypertension. In other words, the lower the AT/ET, the more severe the pulmonary hypertension.

In a measurement of pulmonary artery blood flow, RVOT AT is mainly composed of an acceleration time (AT), an ejection time (ET), and a deceleration time (DT). When the resistance of the pulmonary blood vessels increases, the acceleration time becomes shorter. This index is an echocardiographic index that may evaluate how severe pulmonary hypertension is. The lower the RVOT AT, the shorter the acceleration time, which means a severe pulmonary hypertension status.

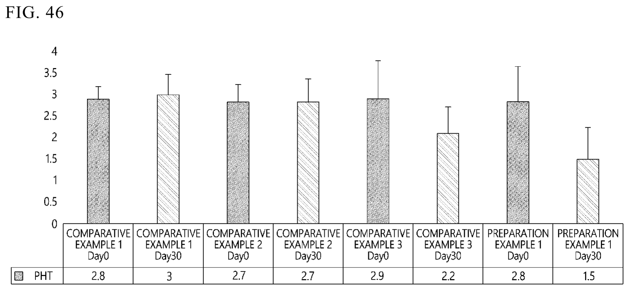

The results of the cardiac ultrasound test were illustrated in Table 15 and FIG. 46. In the evaluation results in Table 15 and FIG. 46, severity of pulmonary hypertension was evaluated by comprehensively evaluating results of TR velocity, PR velocity, RVOT ET/AT, and RVOT AT.

**[Table 15]**

| Test index-Test point in time/Test target | | Preparation Example 1 | Comparative Example 1 | Comparative Example 2 | Comparative Example 3 |
|---|---|---|---|---|---|
| Severity of pulmonary hypertension | DAY0 | 2.8 | 2.8 | 2.7 | 2.9 |
| | DAY30 | 1.5 | 3 | 2.7 | 2.2 |

FIG. 46 is a graph illustrating a severity evaluation result of pulmonary hypertension of Example 2. FIG. 46 illustrates changes in severity of pulmonary hypertension evaluated based on evaluation results of TR velocity, PR velocity, RVOT ET/AT, and RVOT AT. Referring to Table 15 and FIG. 46, pulmonary hypertension becomes worse than that before administration in the group to which the composition of Comparative Example 1 is administered, and a significant difference did not exist before and after administration in the group to which the composition of Comparative Example 2 is administered. A clear decrease in severity of pulmonary hypertension was observed in the groups to which the compositions of Preparation Example 1 and Comparative Example 3 are administered compared to that before administration. In particular, a significant decrease in severity of pulmonary hypertension was observed before and after administration in the group to which the composition of Preparation Example 1 is administered.

### 5. Heart failure index evaluation

As a heart failure index evaluation of Example 2, an ISACHC stage evaluation was performed. In an ISACHC stage evaluation method, a classification index for dogs with heart failure proposed by the International Small Animal Cardiac Heart Council is divided into a total of 5 stages:
1. ISACHC IA asymptomatic sick dog group without cardiomegaly,
2. ISACHC IB asymptomatic sick dog group with cardiomegaly,
3. ISACHC II sick dog group with mild-moderate heart failure,
4. ISACHC IIIA dog group with severe heart failure in which pulmonary edema occurs,
5. ISACHC IIIB dog group with end-stage heart failure having recurrent pulmonary edema.

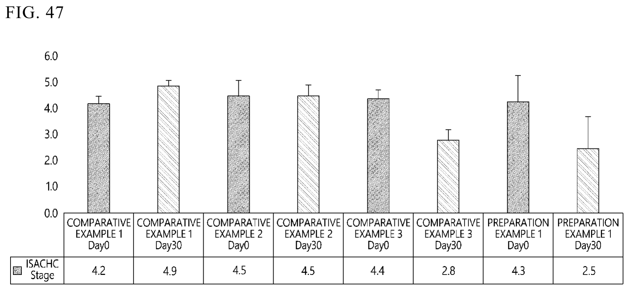

The results of the ISACHC stage evaluation were illustrated in Table 16 and FIG. 47.

**[Table 16]**

| Test index-Test point in time/Test target | Preparation Example 1 | Comparative Example 1 | Comparative Example 2 | Comparative Example 3 |
|---|---|---|---|---|
| ISACHC stage | DAY0 | 4.3 | 4.2 | 4.5 |
| | DAY30 | 2.5 | 4.9 | 4.5 |

FIG.47 is a graph illustrating an evaluation result of an ISACHC stage of Example 2.

Referring to Table 16 and FIG. 47, heart failure becomes worse than that before administration in the group to which the composition of Comparative Example 1 is administered, and a significant difference did not exist before and after administration in the group to which the composition of Comparative Example 2 is administered. A decrease effect of heart failure was observed in the groups to which the compositions of Preparation Example 1 and Comparative Example 3 are administered compared to that before administration, and an improvement effect of heart failure was most clearly observed in the group to which the composition of Preparation Example 1 is administered.

### 6. Test end time

In the present experiment, when pulmonary edema or clinical symptoms worsened within an administration period, the administration was stopped, and a rescue drug (a diuretic or a cardiotonic) was administered instead. The numbers of dogs to which the rescue drug is administered instead within the administration period was compared in the groups to which the compositions of Preparation Example 1 and Comparative Examples 1 to 3 are administered were compared with each other.

In the group to which the composition of Comparative Example 1 is administered, only 2 dogs of a total of 10 sick dogs reached a final experiment end time (DAY 30), 3 dogs of the total of 10 sick dogs required specific drug administration due to worsening of clinical symptoms or occurrence of pulmonary edema only at 1 week of administration, 3 dogs of the total of 10 sick dogs required specific drug administration due to worsening of clinical symptoms or occurrence of pulmonary edema only at 2 weeks of administration, and 2 dogs of the total of 10 sick dogs required stop of test drug administration and required specific rescue drug administration due to worsening of clinical symptoms or occurrence of pulmonary edema only at 3 weeks of administration.

In the group to which the composition of Comparative Example 2 is administered, only 4 dogs of a total of 10 sick dogs reached a final experiment end time (DAY 30), 2 dogs of the total of 10 sick dogs required specific drug administration due to worsening of clinical symptoms or occurrence of pulmonary edema only at 1 week of administration, 2 dogs of the total of 10 sick dogs required specific drug administration due to worsening of clinical symptoms or occurrence of pulmonary edema only at 2 weeks of administration, and 2 dogs of the total of 10 sick dogs required stop of test drug administration and required specific rescue drug administration due to worsening of clinical symptoms or occurrence of pulmonary edema only at 3 weeks of administration.

In the group to which the composition of Comparative Example 3 is administered, 9 dogs of a total of 10 sick dogs reached a final experiment end time (DAY 30), and 1 dog of the total of 10 sick dogs required specific rescue drug administration due to worsening of clinical symptoms or occurrence of pulmonary edema only at 3 weeks of administration.

In contrast, the group to which the composition of Preparation Example 1 is administered, all of 10 sick dogs reached a final experimental end point (DAY 30).

### 7. Conclusion according to experimental results

As a result of the evaluation of Example 2, it could be seen that the composition for preventing and treating heart disease according to an embodiment is effective in preventing and treating heart disease in animals such as dogs and cats, by including pimobendan, which is a cardiotonic ingredient, enalapril, which is a vasodilator, and torsemide and spironolactone, which are diuretic ingredients.

When comparing the number of dogs maintained without the rescue drug until an end of the test (DAY 30) in the present test with each other, it was shown that when the composition according to an embodiment, such as Preparation Example 1 is administered, it is more effective in managing severe heart failure (pulmonary hypertension) to add other types of drugs than to administer pimobendan, which is a cardiotonic, alone. Furthermore, the composition of Comparative Example 2 in which a diuretic is not added was less effective in managing symptoms, and also in the case of the diuretics, it could be seen that the composition of Preparation Example 1 in which two diuretics are mixed with each other is more advantageous in preventing symptom aggravation and improving survival than a single diuretic.

In the group to which the composition of Comparative Example 1 is administered, which is a group in which pimobendan is administered alone, a slight improvement effect of clinical symptoms was observed in dogs with severe heart failure (pulmonary hypertension), but in most dogs with severe heart failure, such an improvement effect was short-term and temporary and symptoms were ultimately aggravated. An improvement effect of the clinical symptoms related to heart failure was better in combination preparations (Preparation Example 1 and Comparative Examples 2 and 3) in which enalapril is additionally administered, and was most obvious particularly in Preparation Example 1 and Comparative Example 3, which are a combination preparation group in which a diuretic is administered together. In particular, an improvement effect of clinical indices was the most excellent in Preparation Example 1, which is a combination preparation of four drugs. In addition, it could be seen that the composition of Preparation Example 1 shows more excellent effects than other compositions of Comparative Examples also in evaluation results in the chest radiography evaluation, the cardiac ultrasound test, and particularly, in the heart failure and the pulmonary hypertension.

The composition according to an embodiment is effective in treating heart failure and pulmonary hypertension of animals by including pimobendan, enalapril, torsemide, and spironolactone, and is easier in managing sick dogs by further including drugs with different mechanisms.

The embodiments of the present disclosure have been described hereinabove with reference to the accompanying drawings, but it will be understood by one of ordinary skill in the art to which the present disclosure pertains that various modifications and alterations may be made without departing from the technical spirit or essential feature of the present disclosure. Therefore, it is to be understood that the embodiments described above are illustrative rather than being restrictive in all aspects.

## Claims

1. A composition for preventing and treating heart disease and pulmonary hypertension of an animal, comprising:
pimobendan;
enalapril;
torsemide; and
spironolactone.

2. The composition for preventing and treating heart disease and pulmonary hypertension of animals of claim 1, wherein the composition comprises 0.5 mg of pimobendan, 1 mg of enalapril, 0.2 mg of torsemide, and 2 mg of spironolactone.

3. The composition for preventing and treating heart disease and pulmonary hypertension of animals of claim 1, wherein based on a weight of a target, a single administration dose of pimobendan is 0.25 mg/kg, a single administration dose of enalapril is 0.5 mg/kg, a single administration dose of torsemide is 0.1 mg/kg, and a single administration dose of spironolactone is 1 mg/kg, and pimobendan, enalapril, torsemide, and spironolactone are administered twice a day.

4. The composition for preventing and treating heart disease and pulmonary hypertension of animals of claim 3, wherein the target is a dog or a cat.

5. A functional preparation for preventing and treating heart disease and pulmonary hypertension of an animal, comprising:
pimobendan;
enalapril;
torsemide; and
spironolactone.

6. The functional preparation for preventing and treating heart disease and pulmonary hypertension of animals of claim 5, wherein the functional preparation comprises 0.5 mg of pimobendan, 1 mg of enalapril, 0.2 mg of torsemide, and 2 mg of spironolactone.

7. The functional preparation for preventing and treating heart disease and pulmonary hypertension of animals of claim 1, wherein based on a weight of a target, a single administration dose of pimobendan is 0.25 mg/kg, a single administration dose of enalapril is 0.5 mg/kg, a single administration dose of torsemide is 0.1 mg/kg, and a single administration dose of spironolactone is 1 mg/kg, and pimobendan, enalapril, torsemide, and spironolactone are administered twice a day.
